(19) **Europäisches Patentamt / European Patent Office / Office européen des brevets**

(11) **EP 2 025 286 B1**

(12) **EUROPÄISCHE PATENTSCHRIFT**

(45) Veröffentlichungstag und Bekanntmachung des Hinweises auf die Patenterteilung:
**09.03.2011 Patentblatt 2011/10**

(51) Int Cl.:
**A61B 5/03** *(2006.01)*     **A61B 5/00** *(2006.01)*

(21) Anmeldenummer: **08168399.7**

(22) Anmeldetag: **27.04.2006**

(54) **Implantierbare Vorrichtung zu Erfassung von intracraniellen Drücken**

Implantable device for recording intracranial pressure

Dispositif implantable destiné à l'enregistrement de pressions intracrâniennes

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.04.2005 DE 102005020569**

(43) Veröffentlichungstag der Anmeldung:
**18.02.2009 Patentblatt 2009/08**

(62) Dokumentnummer(n) der früheren Anmeldung(en) nach Art. 76 EPÜ:
**06724600.9 / 1 887 930**

(73) Patentinhaber:
• **Aesculap AG**
  **78532 Tuttlingen (DE)**
• **Christoph Miethke GmbH & Co. KG**
  **14469 Potsdam (DE)**

(72) Erfinder:
• **Lutze, Theodor**
  **78582 Balgheim (DE)**
• **Schauer, Dirk**
  **10318 Berlin (DE)**
• **Miethke, Christoph**
  **14469 Potsdam (DE)**

(74) Vertreter: **Hoeger, Stellrecht & Partner Patentanwälte**
**Uhlandstrasse 14c**
**70182 Stuttgart (DE)**

(56) Entgegenhaltungen:
**EP-A- 1 312 302     DE-A1- 3 841 429**
**GB-A- 2 043 911     US-A- 3 946 724**

**Beschreibung**

Die Erfindung betrifft eine implantierbare Vorrichtung zur Erfassung von intracraniellen Drücken, wobei eine Druckmesseinrichtung verwendet wird, die in Wirkverbindung mit einem Sensor für eine telemetrische Messwertübertragung steht.

Im Rahmen neurochirurgischer Interventionen kommt der Erfassung des intracraniellen Druckes eine herausragende Bedeutung zu. Die möglichst genaue und einfache minimalinvasive Messung des Hirndruckes zählt bis heute zu den nicht befriedigend gelösten Aufgaben in der Medizintechnik.

Zahlreiche Erfindungen haben sich die Lösung dieses Problems zur Aufgabe gemacht; alle bisher erhältlichen Systeme weisen aber gravierende Nachteile auf, die zu überwinden sich die hier vorgestellte Erfindung zur Aufgabe gemacht hat.

Durchgesetzt in der klinischen Praxis haben sich invasive Meßmethoden, bei denen ein Sensor in den Körper eingebracht wird, wobei über eine Kabelverbindung das Signal an ein externes Gerät zur Darstellung und Auswertung des Messwertes weitergeleitet wird. Häufig geschieht dies im Zusammenspiel mit einer künstlichen Drainageleitung, durch die Hirnwasser extrakorporal abgeleitet werden soll. Kritisch bei solchen Systemen ist die sehr hohe Infektionsgefahr. Lange Verläufe können nur durch sehr aufwendige Infektionsprophylaxe und den mehrfachen Austausch des Drucksensors realisiert werden. Insbesondere bei Patienten mit Hydrocephalus ist aber gerade der klinikferne Verlauf des Hirndruckes nach Implantation eines internen künstlichen Drainagesystems von großem diagnostischem Interesse. Für solche Aufgaben eignen sich Systeme, die das gemessene Signal durch die intakte Haut weiterleiten oder aber die Messung durch die Haut ermöglichen.

In Patent DE 196 38 813 C1 wird ein implantierbarer Drucksensor beschrieben, der mit flexiblen Folienleiterbahnen verbunden ist und von einem Substrat im Bereich des Sensorelementes umgeben ist, das eine höhere mechanische Festigkeit aufweist als die Folienleiterbahn und das zusammen mit dem Sensorelement in eine flexible Masse eingeschlossen ist. Durch den Aufbau soll eine zuverlässige und kostengünstige Messvorrichtung möglich werden, die jedoch wegen der erforderlichen Hautdurchleitung hinsichtlich des Infektionsrisikos keine Minderung bietet. Im Zusammenhang mit der Sensorik wird Bezug genommen auf Patent US 4,738,267, in dem eine Kunststoffkapsel mit Membran verwendet wird, auf die ein Dehnungsmessstreifen aufgebracht wird. Die Verstimmung der Wheatstone-Brücke wird als das Maß für den anliegenden Druck interpretiert. Ein solcher Sensor arbeitet ungenau und weist ein unakzeptabel hohes Driftverhalten auf. Aus diesem Grunde hat er sich als implantierbarer Hirndrucksensor nicht durchgesetzt.

Als Zusatzanmeldung zu DE 196 38 813 C1 wird in der Anmeldung DE 197 05 474 die analoge Technik auch zur telemetrischen Erfassung von intrakorporalen Drücken beschrieben. Allerdings sind hier keine Hinweise gegeben, wie die Biokompatibilität sichergestellt werden soll. Ein solcher Sensor ist bis heute nicht zur Marktreife gelangt.

Ein gleichfalls telemetrisches Verfahren zur Erfassung des intracraniellen Druckes wird in der Patentschrift US 6,113,553 beschrieben. Die hier angemeldeten Ansprüche beziehen sich auf die möglichst driftfreie und langzeitstabile Messung unter Beschreibung des elektronischen Aufbaus. Verwendung findet hier ein kapazitiver Aufbau, wobei der Sensor im Knochen des Patienten versenkt werden soll. Dies ist erforderlich aufgrund des raumgreifenden Aufbaus des Sensors. Inwieweit die tatsächlichen Eigenschaften des Sensorkonzeptes den hohen Anforderungen an die Hirndruckmessung hinsichtlich Genauigkeit und Driftverhalten genügen ist nicht bekannt, da ein solcher Sensor bisher nicht marktverfügbar ist und somit nicht unabhängigen Tests unterzogen werden konnte.

Ein Verfahren zur Erfassung des intracraniellen Druckes ohne Hautdurchleitung ist ebenfalls in Patent US 4,676,255 aus dem Jahre 1987 beschrieben. Die Idee war hier, das Prinzip entspannter Membranen zu nutzen. Ein unter der Haut platzierter Sensor hat seine Nullposition solange intracraniell gegenüber der Umgebung kein positiver oder negativer Differenzdruck anliegt. Steigt oder fällt der intracranielle Druck, bewegt sich der Sensor aus der Nullposition. Von außen wird nun durch die Haut genau der Druck aufgebracht, der nötig ist, um den Sensor wieder in die Nullposition zu bringen. Der dazu notwendige Druck soll dann dem intracraniellen Druck entsprechen. Dieses Verfahren hat sich klinisch nicht durchsetzen können. Gründe hierfür sind die Varianz der Haut von Patient zu Patient, die technisch schwierige und fehlerhafte Erfassung der Nullposition sowie die komplizierte Erzeugung des notwendigen externen Druckkissens.

Wesentlich bessere Möglichkeiten versprechen die telemetrischen Ansätze, bei denen ein extrem kleiner Drucksensor in den Körper eingebracht wird, der über ebenfalls in den Körper eingebrachte Kabel mit einer Spule verbunden ist, über die einerseits der Sensor im Bedarfsfall mit Energie versorgt werden kann und andererseits das gemessene Signal nach außen an eine Empfangseinheit zur Weiterverarbeitung gesendet werden kann.

In Patent DE 198 58 172 wird eine Methode beschrieben, die auf mikrosystemtechnischer Basis den intrakorporalen Druck unmittelbar mittels eines Sensorelementes erfasst. Im Mittelpunkt der Betrachtungen steht die Erfassung des Augeninnendruckes. Das Implantat soll daher möglichst klein und leicht sein. Bei der Verwendung dieser Technologie zur Erfassung des intracraniellen Druckes kommt der Beschichtung des Sensors eine entscheidende Bedeutung zu. In Patentschrift DE 101 56 494 wird ein solcher Sensor beschrieben, bei dem zur Gewährleistung der Biokompatibilität eine Metallschicht sowie zumindest abschnittsweise eine biokompatible Kunststoffschicht vorgesehen ist. Ein solcher Aufbau weist erhebliche Nachteile auf. Eine wie auch immer geartete Beschichtung des Sensorelementes ermöglicht die Beeinträchtigung der Messung wegen der Durchleitung durch eben diese Schicht, deren Eigenschaften sich im Laufe der Zeit verändern können. Die Schicht kann beschädigt werden aufgrund von Krafteinwirkungen von außen. Ebenso kann ein Driftverhalten aufgrund von Alterung insbesondere der Kunststoffschichten problematisch sein.

Um eine homogene und sichere Übertragung des um den Sensor anliegenden Druckes zu gewährleisten wird in Patent EP 1 312 302 A2 eine Technik beschrieben, bei der ein um den Sensor angeordnetes Medium von einer flexiblen Hülle umgeben ist. Wie die Biokompatibilität der flexiblen Hülle gewährleistet werden soll, ist in der Schrift nicht beschrieben. Die in der Anmeldung favorisierte Verwendung von Silikonöl zur optimalen Übertragung des anliegenden Druckes erscheint unter Berücksichtigung von Risikoaspekten problematisch.

Eine Vorrichtung nach dem Oberbegriff von Anspruch 1 ist aus dem Patent US 394 6 724 bekannt.

Der Erfindung liegt die Aufgabe zugrunde, bei einer gattungsgemäßen implantierbaren Vorrichtung einen miniaturisierten Chip zur Erfassung des Absolutdruckes so einzusetzen, dass einerseits die Biokompatibilität des Implantates auch langfristig sichergestellt ist und dass andererseits eine möglichst driftfreie und hochgenaue Messung erfolgen kann.

Diese Aufgabe wird bei einer implantierbaren Vorrichtung der eingangs beschriebenen Art erfindungsgemäß durch die in den Patentansprüchen angegebenen Merkmale gelöst.

Der auf einem Mikrochip angeordnete und in diesen integrierte Drucksensor ist durch die Anordnung in einem starren Gehäuse, das hermetisch abgeschlossen ist, bestmöglich gegen die Umgebung geschützt, und auch die Umgebung ist gegen den Austritt von gefährlichen Substanzen geschützt. Durch die Verwendung einer sehr dünnen Membrane kann der Druck der Hirnflüssigkeit auf den Innenraum des starren Gehäuses übertragen werden, und in diesem Innenraum wird die druckabhängige Bewegung der Membran über ein Übertragungsmittel auf die Druckmesseinrichtung übertragen, so dass eine zuverlässige und sehr unmittelbare Erfassung der Druckschwankungen der umgebenden Hirnflüssigkeit möglich wird.

Insbesondere werden dadurch Schwierigkeiten bei der Passivierung des elektronisch arbeitenden Sensors überwunden, die insbesondere in der Sicherheit des Schutzes im Hinblick auf Alterung oder Beschädigung, in der Beeinträchtigung der Druckübertragung durch die aufgebrachte Schutzschicht und in der sich aus nach der Implantation im Laufe der Zeit einstellenden Materialänderungen ergebenden unkalkulierbaren Drift gesehen werden.

Als Übertragungsmittel wird Luft oder ein spezielles Gas oder eine Flüssigkeit verwendet, wobei dieses Übertragungsmittel eine Kammer im Inneren des starren Gehäuses ausfüllt.

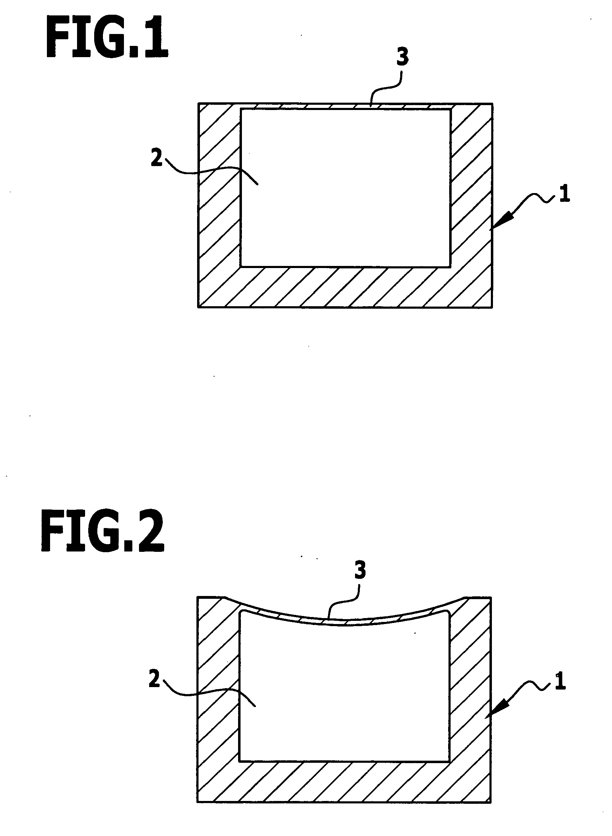

Bei der Verwendung eines Gases als Füllmedium der Kammer lässt sich die Arbeitsweise der Vorrichtung mit Hilfe der idealen Gasgleichung einfach und genau beschreiben. In Figur 1 ist ein zylindrischer Behälter dargestellt, dessen Boden und zylindrische Seitenwand sehr dick ausgelegt sind, dessen Deckel aber sehr dünnwandig als Membrane ausgelegt ist. Es gilt für konstante Temperaturverhältnisse

$$(1) \quad p*V = constant$$

Ändert sich der Druck außerhalb des Behälters, kommt es zu einer Verschiebung der Membran, die sich berechnen lässt und bestimmt wird vom Volumen V1 im Behälter, der Eigenschaft der Membran und dem Wert der außen wirkenden Druckänderung. Figur 2 zeigt eine mögliche Verschiebung einer solchen Membran für den Fall des externen Druckanstieges, wobei aufgrund der durch die Wölbung entstehenden Spannungen in der Membrane die Drücke innerhalb und außerhalb des Behälters unterschiedlich sein können. Allerdings gibt es zu jeder Membranstellung eine charakteristische Drucksituation im Behälter, die einem extern anliegenden Druck entspricht. Durch die Messung des Drucks in dem Behälter kann man also auf den äußeren Druck rückschließen. Die Absolutbewegung der Membrane ist nicht linear zu der anliegenden Druckdifferenz. Will man durch die Druckmessung in einem Behälter eine indirekte Druckmessung durchführen, so sollte die Kammer für den am häufigsten auftretenden Druck eine entspannte Membrane aufweisen wie in Figur 1 dargestellt. Je weniger es nun bei äußeren Druckschwankungen zu Spannungen in der Membrane kommt, desto genauer wird der Druck von außen nach innen übertragen und desto genauer wird die Messung. Im besten Falle erreicht der zu messende Druck keine Werte, bei denen es in der Membran zu wesentlichen Spannungen kommt.

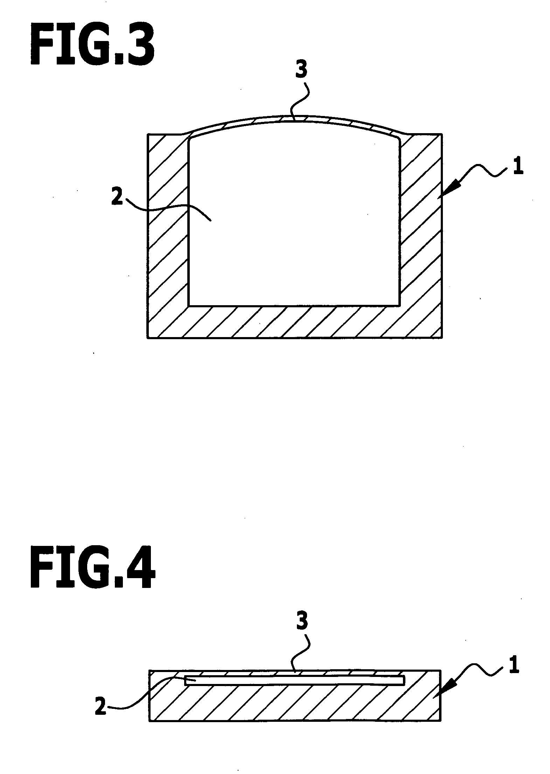

Figur 3 zeigt die Verschiebung für den Fall des Abfalls des extern anliegenden Druckes. Je nach Eigenschaft und Form der Membrane kommt es zu keiner oder nur einer sehr geringen Spannungsänderung innerhalb der Membran. Es gilt das Prinzip der entspannten Membranen, bei dem sich der Druck unter der Voraussetzung, dass die Membrane keine Spannungen aufnimmt, auf beiden Seiten der Membrane auf den gleichen Wert einstellt. Dieses Prinzip ist in der Technik bekannt und wird vielfach genutzt. Bisher ist allerdings nicht erkannt worden, dass sich dieses Prinzip ideal zur Messung von Körperdrücken auch für lange Zeiträume sicher und driftfrei auch für miniaturisierte Mikrochip-Sensoren nutzen lässt, die auf Siliziumbasis hergestellt werden und ihre Leistungsfähigkeit in vielen technischen Anwendungen überzeugend nachweisen konnten.

3

Um eine möglichst effiziente direkte Druckübertragung von außen nach innen zu realisieren, obwohl die metallische Membrane (idealerweise aus Titanblech oder aus einer Titanfolie) vergleichsweise steif ist, sollte das im Sensor zur Verfügung stehende Luftvolumen bei Umgebungsdruck möglichst gering sein. Figur 1 zeigt einen mit Gas (Luft) befüllten Behälter, dessen Innendruck exakt dem Außendruck entspricht, ohne dass die Membrane gewölbt wird. Figur 3 zeigt ebendiesen Behälter, nun jedoch für einen geringeren äußeren Druck: die Membran ist nach außen gestülpt. In der Folge sinkt auch der Druck in dem Behälter. Figur 4 zeigt einen Behälter, bei dem das Luftvolumen minimal klein ausgelegt ist. Da aber in diesem Fall die Membrane genauso ausgelegt ist wie in Figur 1 bis Figur 3, ist das theoretisch verdrängbare Luftvolumen im Sensor, bei dem es noch zu keinen nennenswerten Spannungen in der Membrane kommt, exakt so groß wie in den Fällen mit großem Behältervolumen (Figur 1 bis Figur 3). Die Membran kann nun aufgrund des sehr kleinen inneren Luftvolumens den äußeren Druck ohne große Membranbewegung auf die Behälterkammer übertragen. Um für diesen Behälter auch bei großen Änderungen des äußeren eine Druckgleichheit zwischen Innen und Außen herzustellen, ist eine nur sehr kleine Membranverschiebung notwendig. Aufgrund der Kleinheit der Luftkammer im Behälter gemäß Figur 4 gilt die Darstellung für alle drei Fälle, wie sie in Figur 1 bis Figur 3 dargestellt und beschrieben sind. Die Membranverschiebung ist kaum sichtbar. Bei der Hirndruckmessung sind Druckschwankungen von wenigen cm Wassersäule von therapeutischem Interesse. Die Absolutwerte dieser Druckschwankungen liegen immer im Bereich der atmosphärischen Druckschwankungen, also in etwa bei 10 m Wassersäule +/- 1 m Wassersäule. Sensoren, die beispielsweise im Hochgebirge verwendet werden sollen, müssten also dementsprechend anders ausgelegt werden. Je genauer der normale Umgebungsdruck im Einsatzfall eingeschränkt werden kann, desto präziser können die Druckaufnehmer arbeiten, da der relevante Absolutdruckbereich weiter eingeschränkt werden kann; Messungen des intrakorporalen absoluten Druckes sind dann für außerhalb des Bereiches liegende Druckwerte nicht möglich oder fehlerhaft.

Zur Realisierung eines idealen Drucksensors für die Messung des Hirndrucks wird sensorseitig nur wenig mehr Luftvolumen verwendet, als bei einem Druckgradienten von außen nach innen in Höhe von maximal 100 cm Wassersäule, vorzugsweise auch nur 50 cm Wassersäule, durch die Membranverschiebung verdrängt wird. Der Raum im Inneren des Sensors ist dabei so zu gestalten, dass der Membranbewegung keine Hindernisse durch Sensorkomponenten entgegengestellt werden. Ein Anstieg des zu messenden Drucks auf den maximal zulässigen Wert führt zu einer Membranbewegung, die eine Berührung der Membran mit der eigentlichen Sensoreinheit gerade eben noch nicht zulässt, bei weiterem Anstieg des Druckes kommt es zu einer Berührung.

Insbesondere für große Druckbereiche sind auch Ausführungsformen vorgesehen, bei denen es nicht ausschließlich zu einer spannungsfreien oder spannungsarmen Verschiebung der Membran kommt. Hier ist an Sensoren zu denken, die sowohl bei extrem niedrigem als auch extrem hohem zu messenden Druck einsetzbar sind. In diesem Fall zeigt das Signal des Druckaufnehmers keinen einheitlichen Verlauf. Der Sensor verändert seine Charakteristik in Abhängigkeit vom Spannungszustand in der Membran, die wiederum vom anliegenden Druck abhängt. Solche Sensoren weisen eine individuelle Kennlinie auf, die es ermöglichen, einem gemessenen Druck innerhalb des Sensors einen außen anliegenden Druck zuzuordnen. Diese Zuordnung kann durch Erkennung des Sensors vom äußeren Lesegerät geschehen, das dann den dem übermittelten Signal entsprechenden Druckwert kennt.

Das verwendete Gas kann beispielsweise ein Gas aus der Gruppe der Edelgase sein.

Es ist vorteilhaft, wenn bei Verwendung eines Gases als Übertragungsmittel dessen Gasvolumen kleiner ist als ein Kubikmillimeter, vorzugsweise kleiner als 0,1 Kubikmillimeter.

Bei einer bevorzugten Ausführungsform ist der Gehäuseinnenraum bis auf das vorgesehene Gas- oder Flüssigkeitsvolumen mit einem Gas verdrängenden Füllmaterial ausgefüllt.

Dabei kann es sich beispielsweise um ein Kunststoffmaterial, ein Keramikmaterial oder ein metallisches Material handeln.

Insbesondere ist es vorteilhaft, wenn das Füllmaterial an den drucksensiblen Flächen des Mikrochips eine kleinstvolumige Druckkammer nebst Zuleitungskanal freilässt und wenn das Füllmaterial unterhalb der Membran einen Gehäusehohlraum als Druckkammer freilässt und wenn die beiden Druckkammern durch eine kleinvolumige Leitung verbunden sind.

Das Übertragungsmittel kann auch ein hochviskoses Öl sein.

Das starre Gehäuse kann aus allen Materialien bestehen, die biokompatibel sind. Besonders vorteilhaft ist die Ausgestaltung aus Keramik, aus einem biokompatiblen Kunststoff, wie beispielsweise Polyetheretherketon oder Polyetherketonketon oder die Ausbildung als metallisches Gehäuse, wobei als Metall vorzugsweise Titan oder eine Titanlegierung zum Einsatz kommt.

Die Membran besteht aus Metall, insbesondere aus Titan oder einer Titanlegierung.

Dabei ist es vorteilhaft, wenn die Membrane eine Dicke von weniger als 0,05 Millimetern, vorzugsweise von weniger als 0,01 Millimeter, und insbesondere von etwa 0,005 Millimetern aufweist.

Je nach Ausführungsform kann die Membran eine nachgiebige Fläche zwischen 1 mm$^2$ und 100 mm$^2$ aufweisen, insbesondere von etwa 4 mm$^2$.

Es ist günstig, wenn die Membrane mit dem Gehäuse verschweißt ist.

Beispielsweise kann vorgesehen sein, dass die Membrane mit einem Blechrahmen versehen ist und mit dem Rahmen am Gehäuse verschweißt ist.

Bei einer speziellen Ausführungsform mit einem rohrförmigen Gehäuse kann der Rahmen durch eine Hülse gebildet sein, die auf das rohrförmige Gehäuse schließend aufschiebbar ist.

Diese Hülse kann gleichzeitig mit der Membrane am rohrförmigen Gehäuse entlang der Außenkante der Hülse verschweißt werden, wobei ein Loch in der Gehäusewand genau so verschlossen wird, dass nicht die Hülse, wohl aber die Membrane über dem Loch zu liegen kommt und dort fixiert wird.

Bei einer anderen bevorzugten Ausführungsform ist vorgesehen, dass die Membran einstückig mit dem Gehäuse ausgebildet ist. Dadurch ergibt sich eine maximaler Sicherheit gegen Undichtigkeit.

Die Membrane kann unterschiedlich dicke Bereiche aufweisen. Insbesondere kann vorgesehen sein, dass die Membrane in ihren Randbereichen dicker ist als in ihrem zentralen Bereich. Dadurch wird im Übergangsbereich zwischen Membran und Gehäuse eine hohe Festigkeit erreicht, durch die eine Undichtigkeit in diesem Bereich mit Sicherheit vermieden werden kann. Bei einer bevorzugten Ausführungsform ist vorgesehen, dass der Mikrochip neben mindestens einem Drucksensor mindestens einen weiteren Sensor aufweist. Es kann sich dabei beispielsweise um einen Temperatursensor handeln.

Weiterhin ist es günstig, wenn der Mikrochip neben dem Drucksensor und neben gegebenenfalls weiteren Sensoren einen Analog-Digital-Wandler umfasst, der die analogen elektrischen Signale der Sensoren in digitale Signale umwandelt. Auf diese Weise wird sichergestellt, dass die Signale vom Sensor in digitaler Form an eine extrakorporale Auswerteeinheit übertragen werden, so dass die Störanfälligkeit gegenüber der Übertragung von analogen Signalen ganz erheblich herabgesetzt wird. Der Analog-Digital-Wandler kann auf dem Mikrochip ebenso integriert sein wie der Sensor und wie gegebenenfalls andere elektronische Funktionseinheiten, beispielsweise Einheiten zur eindeutigen Identifikation des Mikrochips oder Einheiten, die die digitalen Signale für die Übertragung über einen hochfrequenten Träger vorbereiten.

Durch die Integration dieser Funktionen in einem Mikrochip ist es möglich, eine sehr kleinbauende Vorrichtung zu konstruieren, so dass diese Vorrichtung ohne Schwierigkeiten am gewünschten Ort platziert werden kann.

Bei einer besonders bevorzugten Ausführungsform ist vorgesehen, dass der Mikrochip mit einer Energie- und Signalübertragungsleitung verbunden ist, die zu einem extrakorporalen Datenverarbeitungsgerät oder zu einer Übertragungsspule führt. Energie kann somit dem Mikrochip extern zugeführt werden, es sind keine Energiespeicher in der Vorrichtung notwendig, dadurch werden einerseits die Lebensdauer erhöht und andererseits ist dadurch sichergestellt, dass die implantierbare Vorrichtung sehr klein sein kann.

Zusätzlich zu dem Mikrochip können bei allen Ausführungsformen im Gehäuse noch weitere elektronische Bauelemente angeordnet werden, beispielsweise Dioden und Kondensatoren zur Leistungsbegrenzung und Leistungspufferung. Es ist dabei vorteilhaft, wenn im Gehäuse ein Träger angeordnet ist, auf den sowohl der Mikrochip als auch diese elektronischen Bauelemente aufgesetzt sind und der auch die Verbindungsleitungen zwischen dem Mikrochip, den zusätzlichen elektronischen Bauelementen und der Energie- und Signalübertragungsleitung aufnimmt.

Grundsätzlich ist es möglich, die Übertragungsspule in dem starren Gehäuse anzuordnen, in den meisten Fällen ist es aber von Vorteil, die Übertragungsspule außerhalb des starren Gehäuses anzuordnen, so dass das starre Gehäuse nur den Mikrochip und die unbedingt notwendigen elektronischen Einheiten darauf umschließt und entsprechend klein aufgebaut sein kann.

Es ist vorteilhaft, wenn in dem Gehäuse alle elektrischen und elektronischen Bauelemente und sonstige nicht-biokompatible Komponenten untergebracht sind, da dadurch diese Bauelemente gegenüber der Umgebung hermetisch abgeschlossen sind. Allenfalls die Übertragungsspule kann bei bestimmten Ausführungsbeispielen außerhalb des Gehäuses angeordnet werden, und diese lässt sich biokompatibel ausgestalten. Beispielsweise kann die Spule aus Gold, Platin oder Silber bestehen und atoxisch und biokompatibel ummantelt sein.

Die Energie- und Signalübertragungsleitung wird vorteilhafterweise durch eine hermetisch abgedichtete Durchführung aus dem starren Gehäuse herausgeführt. Dadurch wird sichergestellt, dass der Innenraum des Gehäuses gegenüber dem Außenraum absolut dicht verschlossen ist, trotzdem kann dem Mikrochip Energie zugeführt werden und die vom Mikrochip erzeugten digitalen Signale können zu einer Auswerteeinheit übertragen werden. Eine solche hermetische Durchführung ist bei der Anordnung der Übertragungsspule im Inneren des starren Gehäuses natürlich nur für die Energieübertragung notwendig, wenn die Übertragungsspule jedoch außerhalb des starren Gehäuses angeordnet wird, muss über eine solche Durchführung sowohl die Energieübertragungsleitung als auch die Signalübertragungsleitung hindurchgeführt werden. Es kann sich dabei um getrennte Leitungen handeln oder aber auch um eine gemeinsame Leitung.

Günstig ist es, wenn in der Energie- und Signalübertragungsleitung eine lösbare Kupplung angeordnet ist, insbesondere in Form eines Steckkontaktes. Dabei ist in der Regel vorgesehen, dass dieser Steckkontakt ebenfalls hermetisch gegenüber der Umgebung abgedichtet ist.

In anderen Fällen wird die hermetische Durchführung im Gehäuse mit der Energie- und Signalübertragungsleitung dauerhaft verbunden, beispielsweise durch Löten, Schweißen, Leitkleben oder Crimpen.

Es ist weiterhin vorteilhaft, wenn das starre Gehäuse teilweise mit einer Kunststoffummantelung oder einer Kunststoffabdeckung versehen ist, die allerdings zumindest die Fläche der Membran frei lässt. Eine solche Kunststoffummantelung oder Kunststoffabdeckung schützt das Gehäuse zusätzlich, auch das umgebende Gewebe wird dadurch zusätzlich geschützt.

Die Hirndruckmessung kann an unterschiedlichen Messorten im Gehirn erfolgen, und entsprechend den unterschiedlichen Messorten ergeben sich auch unterschiedliche Ausgestaltungen für das starre Gehäuse.

Bei einer ersten bevorzugten Ausführungsform ist vorgesehen, dass das starre Gehäuse rohrförmig ausgebildet ist.

Bei einem solchen rohrförmigen Gehäuse kann in der Gehäusewand ein Fenster vorgesehen sein, das durch die Membran verschlossen ist.

Besonders günstig ist es, wenn der Durchmesser des Gehäuses zwischen 2 mm und 3,5 mm liegt, insbesondere zwischen 2,5 mm und 3 mm. Ein solches Gehäuse ist insbesondere für die intraventrikuläre Druckmessung geeignet, der Außendurchmesser entspricht einem typischen, in der Hydrocephalus-Therapie standardmäßig verwendeten Ventrikelkatheter.

Dabei kann die Länge des Gehäuses zwischen 10 mm und 30 mm liegen, insbesondere bei etwa 20 mm. Es handelt sich also um ein sehr kleines Gehäuse, das auch in tieferliegenden Bereichen des Gehirns ohne Schwierigkeiten angeordnet werden kann und das dann über die Energie- und Signalübertragungsleitung mit einer Übertragungsspule oder mit der Auswerteeinheit in Verbindung steht.

Dabei ist es vorteilhaft, wenn der Drucksensor etwa in der Mitte der Längenausdehnung des Gehäuses angeordnet ist.

Bei einer anderen Ausführungsform ist vorgesehen, dass die Länge des rohrförmigen Gehäuses zwischen 80 mm und 120 mm liegt, insbesondere bei etwa 100 mm. Bei einer solchen Ausgestaltung führt das rohrförmige Gehäuse von der Messstelle bis an die Außenseite des Schädels, so dass eine hermetisch durch die Gehäusewand hindurchgeführte Energie- und Signalübertragungsleitung nicht unbedingt notwendig ist.

Bei einer solchen Ausgestaltung ist es vorteilhaft, wenn der Mikrochip und die Membran an einem Ende des rohrförmigen Gehäuses angeordnet sind und eine Übertragungsspule am gegenüberliegenden Ende. Diese liegt dann vorzugsweise außerhalb des Schädelknochens.

Bei einer anderen bevorzugten Ausgestaltung kann das starre Gehäuse eine außenseitig an die Membran anschließende, abgeschlossene Flüssigkeitskammer aufweisen, die mit einer Zufuhrleitung für Flüssigkeit verbunden ist. Bei einer solchen Ausgestaltung wird vom Drucksensor über die Membran der Druck der Flüssigkeit in der abgeschlossenen Flüssigkeitskammer bestimmt. Es kann sich dabei unmittelbar um Hirnflüssigkeit handeln oder aber um eine Messflüssigkeit, die sich in der abgeschlossenen Flüssigkeitskammer befindet und die auf andere Weise vom Druck der Hirnflüssigkeit beaufschlagt wird.

Die Flüssigkeitskammer kann zusätzlich eine Ableitungsleitung für Flüssigkeit aufweisen. Dies ist insbesondere dann von Bedeutung, wenn durch die Flüssigkeitskammer Hirnflüssigkeit hindurchgeleitet wird, die dann über die Ableitungsleitung aus dem Hirnraum entfernt wird.

Bei einer solchen Ausgestaltung ist es günstig, wenn das starre Gehäuse die Form einer flachen Dose aufweist mit einer oberen, den Mikrochip und das Übertragungsmittel aufnehmenden Messkammer und einem unteren, die Flüssigkeitskammer ausbildenden Bereich. Ein solches Gehäuse in Form einer flachen Dose kann außen auf den Schädelknochen aufgelegt werden, und zwar entweder über einem Bohrloch im Schädelknochen oder unmittelbar daneben.

Es ist vorteilhaft, wenn die Membran den Innenraum des Gehäuses in die Messkammer und die Flüssigkeitskammer unterteilt, dadurch erstreckt sich die Membran über eine sehr große Fläche und reagiert entsprechend empfindlich auf Druckänderungen der Flüssigkeit in der Flüssigkeitskammer.

Die Zufuhrleitung für die Flüssigkeit kann relativ zu einer unteren Begrenzungswand der Flüssigkeitskammer im Wesentlichen senkrecht verlaufen. Dies ist insbesondere dann vorteilhaft, wenn das Gehäuse unmittelbar auf ein Bohrloch im Schädelknochen aufgesetzt wird, die Zufuhrleitung kann dann durch dieses Bohrloch hindurchtreten.

Beispielsweise kann die Zufuhrleitung für die Flüssigkeit relativ zu einer unteren Begrenzungswand der Flüssigkeitskammer im Wesentlichen mittig in die Flüssigkeitskammer eintreten.

Bei einer anderen Ausführungsform ist vorgesehen, dass die Zufuhrleitung für die Flüssigkeit relativ zu einer unteren Begrenzungswand der Flüssigkeitskammer im Wesentlichen parallel verläuft.

Auch die Ableitungsleitung für die Hirnflüssigkeit kann parallel zu der unteren Begrenzungswand verlaufen. Bei einer solchen Ausgestaltung kann das Gehäuse auch neben dem Bohrloch in der Schädeldecke angeordnet werden, die Zufuhrleitung wird dann durch das Bohrloch hindurch geführt und parallel zum Schädelknochen in die Flüssigkeitskammer eingeleitet.

Günstig ist es, wenn in der Ableitungsleitung ein Rückschlagventil angeordnet ist.

Die Ableitungsleitung kann in ein Reservoir einmünden, aus dem beispielsweise Flüssigkeit rückgespült werden kann, um die Flüssigkeitsleitungen zu reinigen.

Es ist günstig, wenn die Zufuhrleitung mit einem Verlängerungsröhrchen verbunden ist, das an seinem der Flüssigkeitskammer abgewandten Ende offen ist. Ein solches Verlängerungsröhrchen wirkt als Ventrikelkatheter und kann Hirnflüssigkeit aus dem Messbereich direkt in die Flüssigkeitskammer einleiten, mit einer solchen Anordnung lässt sich der Hirndruck am Eingangsbereich des Verlängerungsröhrchens bestimmen.

Bei einer anderen Ausführungsform ist vorgesehen, dass die Zufuhrleitung mit einem Verlängerungsröhrchen verbunden ist, welches an seinem der Flüssigkeitskammer abgewandten Ende mittels einer flexiblen Membran verschlossen ist. Das Verlängerungsröhrchen und die Flüssigkeitskammer sind mit einer Flüssigkeit oder einem Gas gefüllt und bilden ein abgeschlossenes Volumen, das als Druckübertragungsmedium zwischen der das Verlängerungsröhrchen verschließenden Membran und der die Messkammer verschließenden Membran fungiert.

Die Wandstärke des starren Gehäuses kann zwischen 0,3 mm und 2 mm liegen, so dass ein verformungsstabiles, starres Gehäuse erhalten wird.

Um diese Verformungsstabilität noch zu erhöhen, kann vorgesehen sein, dass die Wände des starren Gehäuses durch Verstärkungsstrukturen gegen eine Verformung gesichert sind.

Bei einer weiteren bevorzugten Ausführungsform kann die Messkammer in einem Einsatz angeordnet sein, der deckelartig in das Gehäuse einsetzbar ist und dieses verschließt, wobei der Einsatz eine die Messkammer gegenüber dem die Flüssigkeitskammer bildenden Innenraum des Gehäuses abtrennende Membran trägt. Der Einsatz mit der Membran und der abgetrennten Messkammer bildet somit ein separates Bauteil, das in das Gehäuse einsetzbar ist und das durch das Einsetzen einerseits das Gehäuse verschließt und andererseits die Flüssigkeitskammer abtrennt.

Die Membran kann eine Metallfolie sein, die mit dem die Messkammer aufnehmenden Bauteil verlötet oder verschweißt ist. Dies ist auch dann möglich, wenn die Membran nicht an einem separaten Einsatz gehalten ist, sondern an einem Teil des starren Gehäuses selbst.

Durch die Verlötung und Verschweißung ergibt sich eine dauerhafte und sichere Abdichtung der Messkammer.

Gemäß einer bevorzugten Ausführungsform kann vorgesehen sein, dass das die Messkammer aufnehmende Bauteil einen ebenen Rand aufweist, an dem die Membran flächig anliegt, dass auf der dem Bauteil gegenüberliegenden Seite ein ringförmiges Anlageelement dem ebenen Rand gegenüberliegend angeordnet ist und dass die Membran sowohl mit dem Bauteil als auch mit dem Anlageelement verlötet oder verschweißt wird. Das Anlageelement und das Bauteil nehmen also die Membran sandwichartig zwischen sich auf und ermöglichen eine Verlötung oder Verschweißung der Membran sowohl mit dem Bauteil als auch dem Anlageelement, so dass die Verbindungsstelle nach außen hin auch mechanisch gesichert ist.

Das Bauteil kann aus Metall bestehen, vorzugsweise aus Titan oder einer Titanlegierung.

Ebenso kann das Anlageelement aus Metall bestehen, insbesondere aus Titan oder einer Titanlegierung.

Die Membran besteht aus Metall , insbesondere aus Titan oder einer Titanlegierung.

Es ist dabei günstig, wenn die Dicke der Membran zwischen 1/100 mm und 5/100 mm liegt, vorzugsweise in der Größenordnung von 2 bis 3/100 mm.

Das Anlageelement kann vorzugsweise eine Höhe zwischen 3/10 und 8/10 mm aufweisen, insbesondere in der Größenordnung von 5/10 mm.

Die vorstehend beschriebenen Merkmale sind besonders vorteilhaft in ihrer Kombination, die Erfindung bezieht sich aber auch auf Ausgestaltungen, bei denen nur einzelne dieser Merkmale verwirklicht sind oder bei denen nur ein Teil dieser Merkmale in Kombination verwirklicht wird.

Die nachfolgende Beschreibung bevorzugter Ausführungsformen der Erfindung dient im Zusammenhang mit der Zeichnung der näheren Erläuterung. Es zeigen:

Figur 1:  eine schematische Ansicht eines starren Gehäuses zur Aufnahme eines mit einem Drucksensor versehenen Mikrochips und einer das Gehäuse einseitig verschließenden dünnen, flexiblen Membran in einer Neutralstellung;

Figur 2:  eine Ansicht ähnlich Figur 1 mit der Membran in einer eingedrückten Stellung;

Figur 3:  eine Ansicht ähnlich Figur 1 mit der Membran in einer ausgebauchten Stellung;

Figur 4:  eine Ansicht ähnlich Figur 1 mit einem von dem Gehäuse umgebenen sehr kleinvolumigen Innenraum;

Figur 5:  eine schematische Ansicht eines starren, mit einer Membran verschlossenen Gehäuses mit einem darin angeordneten Mikrochip mit unterschiedlichen Funktionsbereichen;

Figur 5a:  eine Ansicht ähnlich Figur 5 mit einem den Mikrochip und zusätzliche elektronische Bauelemente aufnehmenden Träger;

Figur 6:  eine schematische Längsschnittansicht eines rohrförmigen Gehäuses mit einer Membran und einem im Gehäuse angeordneten Mikrochip;

Figur 7:  eine vergrößerte Detailansicht des Gehäuses der Figur 6 im Bereich der Membran;

Figur 8:  ein hülsenförmiger Rahmen zum Aufschieben auf das rohrförmige Gehäuse der Figur 6;

Figur 9:  eine perspektivische Ansicht des unteren Endes des Gehäuses der Figur 6 mit aufgeschobenem, hülsenförmigem Rahmen;

Figur 10: eine schematische Längsschnittansicht des röhrchenförmigen Gehäuses der Figur 6 mit darin gelagerter Übertragungsspule;

Figur 11: eine schematische Darstellung eines im Inneren des Gehirns platzierten starren Gehäuses mit darin angeordnetem Mikrochip und einer auf dem Schädelknochen angeordneten Übertragungsspule;

Figur 12: eine schematische Detailansicht der implantierbaren Vorrichtung der Figur 11;

Figur 13: eine schematische Ansicht einer Verbindungseinrichtung einer Energie- und Signalleitung zwischen dem starren Gehäuse und der Übertragungsspule bei der implantierbaren Vorrichtung der Figur 11;

Figur 14: eine perspektivische Teilschnittansicht eines bevorzugten Ausführungsbeispiels eines starren Gehäuses mit darin aufgenommenem Mikrochip;

Figur 15: eine Schnittansicht durch eine Übertragungsspule;

Figur 16: ein Handhabungswerkzeug für die Implantation des starren Gehäuses in das Gehirn vor dem Einführen des starren Gehäuses in das Innere des Gehirns;

Figur 17: eine Darstellung ähnlich Figur 16 beim Vorschieben des starren Gehäuses in das Gehirn;

Figur 18: eine Darstellung ähnlich Figur 16 nach der Platzierung des starren Gehäuses im Gehirn und beim Zurückziehen des Handhabungsinstrumentes;

Figur 19: eine Ansicht ähnlich Figur 16 mit platziertem starrem Gehäuse und nach dem Herausziehen des Handhabungsinstrumentes;

Figur 20: eine Ansicht ähnlich Figur 11 mit einer außerhalb des Schädelknochens angeordneten starren Gehäuse und einem in das Innere des Gehirns reichenden Verbindungsröhrchen;

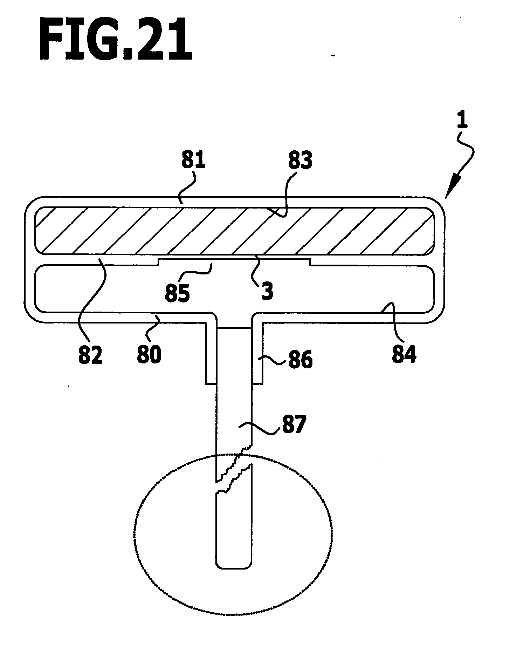

Figur 21: eine schematische Schnittansicht durch ein starres Gehäuse mit einer Messkammer und einer Flüssigkeitskammer;

Figur 22: eine Schnittansicht eines bevorzugten Ausführungsbeispiels eines starren Gehäuses mit einer einen Mikrochip aufnehmenden Messkammer und einer die Flüssigkeitskammer durchsetzenden Flüssigkeitsleitung;

Figur 23: eine Ansicht ähnlich Figur 22 mit einer senkrecht in den Boden des Gehäuses einmündenden Zulaufleitung;

Figur 24: eine schematische Darstellung eines ein Bohrloch in der Schädeldecke überdeckenden starren Gehäuses mit einer katheterförmigen Zulaufleitung;

Figur 25: eine Ansicht ähnlich Figur 23 mit einer das starre Gehäuse überdeckenden Schutzkappe;

Figur 26: eine Ansicht eines starren Gehäuses ähnlich Figur 23, jedoch ohne Ableitleitung;

Figur 27: eine schematische Seitenansicht des starren Gehäuses der Figur 23 mit einem Rückschlagventil in der Ableitleitung und einem mit der Ablaufleitung verbundenen Flüssigkeitsreservoir;

Figur 28: eine schematische Darstellung eines neben einem Bohrloch in der Schädeldecke angeordneten starren Gehäuses und einer durch das Bohrloch in das Gehirn führenden Zufuhrleitung;

Figur 29: eine schematische Seitenansicht ähnlich Figur 21 mit einer durch eine Membran verschlossenen, katheterförmigen Verlängerung;

Figur 30: eine schematische Schnittansicht eines starren Gehäuses ähnlich Figur 23, jedoch ohne Übertragungsspule im Inneren des starren Gehäuses;

Figur 31: eine schematische Darstellung eines ein Bohrloch in der Schädeldecke abdeckenden starren Gehäuses mit einer neben dem starren Gehäuse auf der Schädeldecke aufgelegten Übertragungsspule;

Figur 32: eine Ansicht ähnlich Figur 31 mit einer das starre Gehäuse umgebenden Übertragungsspule;

Figur 33a: eine schematische Querschnittsdarstellung eines Gehäuses mit einem vorbestimmten Wandbereich reduzierter Dicke vor einem Ätzvorgang zur Herstellung einer Membran;

Figur 33b: eine Ansicht ähnlich Figur 33a nach einem Ätzvorgang;

Figur 34a: eine Ansicht ähnlich Figur 33a bei einer anderen Querschnittsgestaltung des vorbestimmten Wandbereiches;

Figur 34b: eine Ansicht ähnlich Figur 34a nach dem Ätzvorgang;

Figur 35a: eine schematische Darstellung eines Teilbereiches des Mikrochips mit einer Silikonbeschichtung vor der endgültigen Positionierung von Mikrochip und Membran;

Figur 35b: eine Ansicht ähnlich Figur 35a nach der endgültigen Positionierung von Mikrochip und Membran mit bereichsweiser Anlage der Silikonbeschichtung an der Membran;

Figur 36a: eine Ansicht ähnlich Figur 35a mit nur bereichsweiser Beschichtung des Mikrochips;

Figur 36b: eine Ansicht ähnlich Figur 35b mit nur bereichsweiser Beschichtung des Mikrochips;

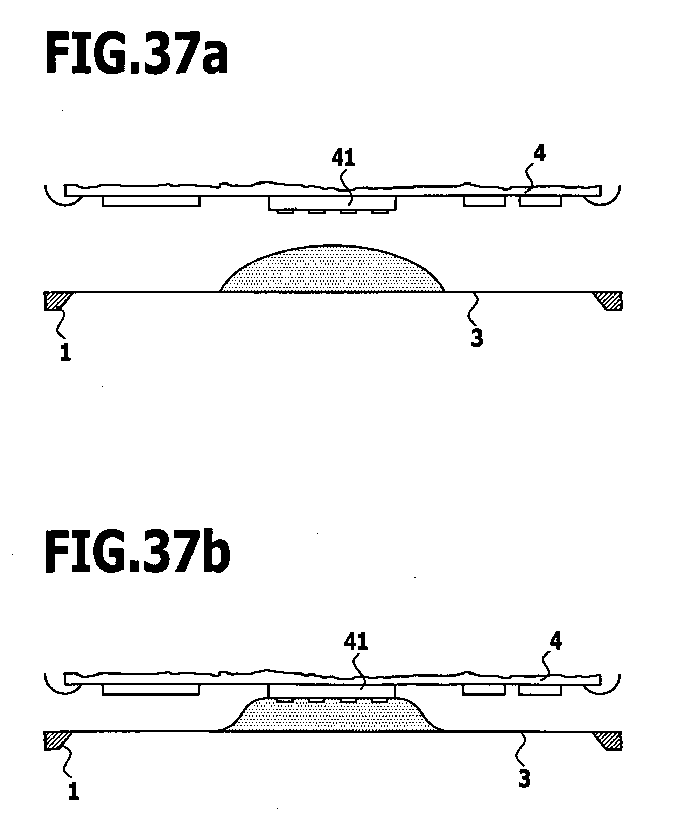

Figur 37a: eine Ansicht ähnlich Figur 35a mit einer Silikonbeschichtung auf der Membran;

Figur 37b: eine Ansicht ähnlich Figur 35b mit einer Silikonbeschichtung auf der Membran;

Figur 38: eine schematische Seitenansicht eines Gehäuses mit einer Membran und einem am Mikrochip gehaltenen und sich an der Membran abstützenden Druckstempel;

Figur 39: eine schematische, perspektivische Ansicht der Anordnung von Mikrochip und Druckstempel gemäß Figur 38;

Figur 40: eine schematische Schnittansicht durch ein Gehäuse mit Membran und einer U-förmigen Bügelfeder zwischen Mikrochip und Membran;

Figur 41: eine schematische Ansicht der Anordnung von Mikrochip und Blattfeder gemäß Figur 40;

Figur 42: eine Ansicht ähnlich Figur 40 bei einem Ausführungsbeispiel mit einem Bügel und zwei Druckstempeln;

Figur 43: eine Ansicht ähnlich Figur 40 mit einem seitliche Stege aufweisenden Bügel;

Figur 44: eine Ansicht der Anordnung aus Mikrochip und Bügel gemäß Figur 43;

Figur 45: eine Schnittansicht durch einen eine Messkammer aufnehmenden Einsatz mit einer die Messkammer verschließenden Membran und

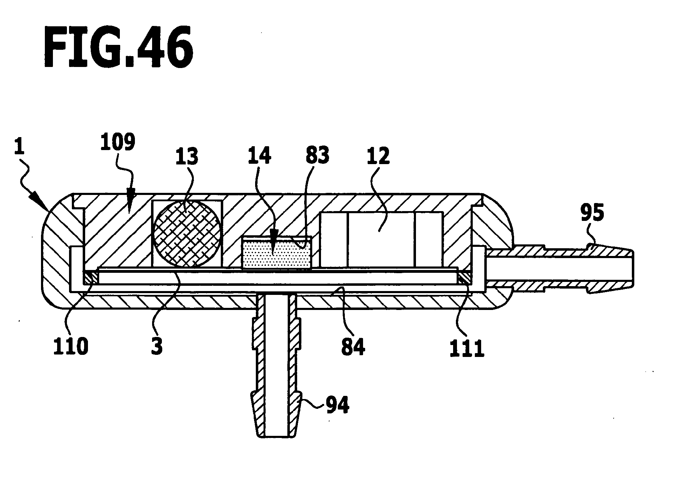

Figur 46: eine Schnittansicht durch ein Gehäuse mit einem in das Gehäuse eingesetzten Einsatz gemäß Figur 45.

Wie bereits erörtert umfasst die implantierbare Vorrichtung zur Bestimmung des Hirndruckes ein starres Gehäuse 1 mit einem Innenraum 2, welcher nach außen durch eine flexible, vorzugsweise elastische Membran 3 verschlossen ist. Das starre Gehäuse ist so ausgebildet, dass es bei den auftretenden Drücken möglichst verformungsfrei ist, es kann beispielsweise aus Keramik, einem biokompatiblen Kunststoff (Polyetheretherketon, Polyetherketonketon) oder aus Metall (Titan, Titanlegierung) bestehen und kann zusätzlich eine innere Verstärkungsstruktur aufweisen, beispielsweise durch den Innenraum durchsetzende Stützen oder durch Verstärkungsrippen am Gehäuse 1, die in der Zeichnung nicht dargestellt sind.

Die Wandstärke des Gehäuses liegt zwischen 0,3 Millimetern und 2 Millimetern, während die Dicke der Membran erheblich geringer ist, beispielsweise in der Größenordnung zwischen 0,005 Millimetern und 0,05 Millimetern.

Zur Herstellung der Membran kann insbesondere von einem einstückigen Gehäuse ausgegangen werden, das durch eine spanabhebende Bearbeitung oder in anderer Weise in einem bestimmten Wandbereich in seiner Dicke reduziert wird. Man erhält dann ein Gehäuse, das im größten Teil verformungsstabile Wände aufweist, lediglich im Bereich des vorbestimmten Wandbereiches wird die Wandstärke durch die mechanische Vorbearbeitung geschwächt.

Anschließend wird die Wand in dem vorbestimmten Wandbereich in ihrer Dicke weiter reduziert, und zwar durch einen chemischen Ätzvorgang oder durch eine elektrolytische Abtragung, bis die gewünschte Dicke der Membran erreicht wird.

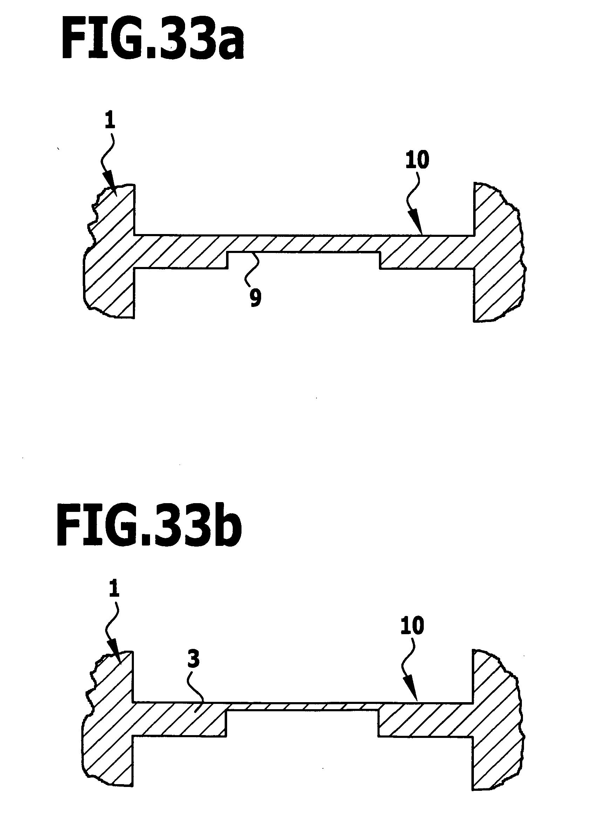

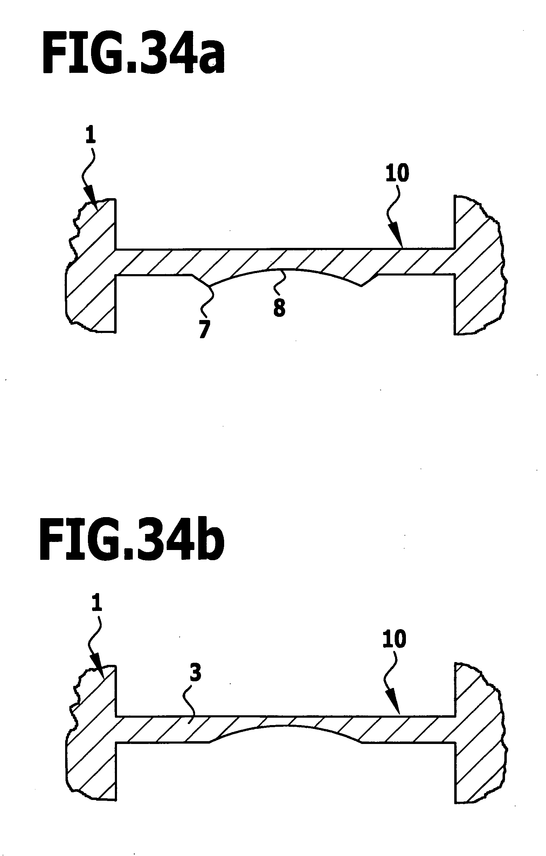

In den Figuren 33a und 34a sind mögliche Geometrien der vorbestimmten Wandbereiche 10 dargestellt, die durch eine spanabhebende Bearbeitung hergestellt werden, beispielsweise durch Fräsen oder Ausdrehen. Dabei wird die verbleibende Dicke des Materials in den vorbestimmten Wandbereichen 10 unterschiedlich ausgeführt. Beim Ausführungsbeispiel der Figur 33a beispielsweise ist im zentralen Wandbereich eine stufige Vertiefung 9 angeordnet, im Ausführungsbeispiel der Figur 34a eine muldenförmige Vertiefung 8 mit erhöhten Rändern 7. Nach dem Ätzvorgang erhält man auf diese Weise eine Membran 3, deren Dicke im zentralen Bereich geringer ist als im Randbereich. Geht man von einer Geometrie gemäß Figur 33a aus, so erhält man nach dem Ätzvorgang einen Querschnitt, wie er in Figur 33b dargestellt ist, also einen Querschnitt mit einer stufigen Vertiefung im zentralen Bereich, geht man von einer Geometrie aus, wie sie in Figur 34a dargestellt ist, erhält man eine Membran mit einer Querschnittsfläche gemäß Figur 34b, also mit einer zentralen Mulde, die stufenfrei in die Membranfläche übergeht.

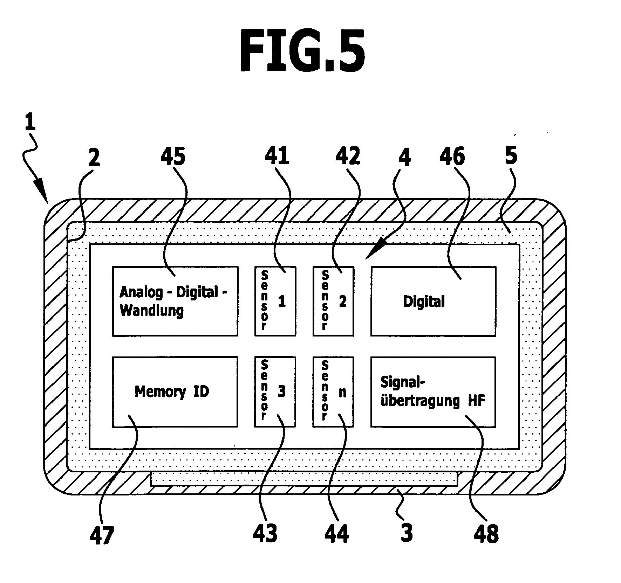

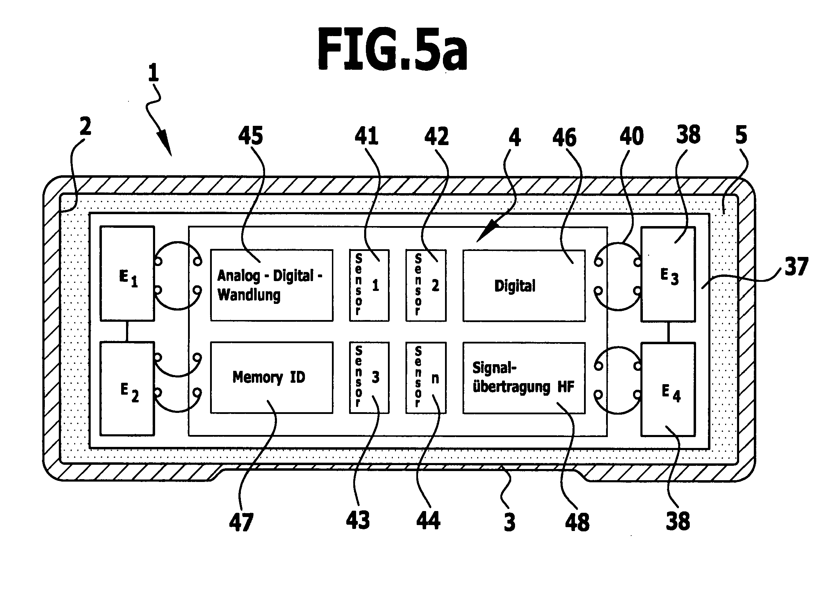

Der Innenraum 2 oder zumindest ein Teil desselben ist mit einem Übertragungsmittel in Form eines Gases oder einer Flüssigkeit gefüllt. Durch dieses Übertragungsmittel werden Druckschwankungen der Umgebung, die zu einer Verformung der Membran 3 führen, auf den Innenraum 2 übertragen und dort u.a. auch auf einen Mikrochip 4, der im Innenraum 2 angeordnet ist (Figur 5 und Figur 5a).

Insbesondere bei der Verwendung eines Gases als Übertragungsmittel ist es günstig, wenn der Innenraum 2 sehr kleinvolumig ausgebildet ist, wie dies in Figur 4 dargestellt ist.

Grundsätzlich sind verschiedene Messorte zur Hirndruckmessung etabliert. In den meisten Fällen empfiehlt sich die intraventrikuläre Messung, für die parenchymatöse, die epidurale oder die subdurale Messung sind ebenfalls entsprechende Ausführungsbeispiele denkbar.

Konkret empfiehlt sich zur intraventrikulären Druckmessung die Verwendung eines Titanröhrchens mit dem Außendurchmesser von etwa 3 mm, was den Dimensionen eines typischen in der Hydrocephalus-Therapie standardmäßig verwendeten Ventrikelkatheters entspricht. Das Gehäuse ist endständig durch eine Halbkugel verschlossen. Möglichst nahe dieser halbrunden Spitze (vorzugsweise etwa 1 bis 3 mm entfernt) wird in die zylindrische Gehäusewand ein Fenster eingebracht, welches mit einer extrem dünnen metallischen Folie wieder verschlossen wird. Die Wand des metallischen Röhrchens, welches aus einem biokompatiblen Material gefertigt wird, weist eine gegenüber der das Fenster abdeckenden Folie etwa 10-fache Stärke auf, sie kann aber auch noch stärker ausgebildet sein. Vorzugsweise beträgt die Stärke der Folie 0,01 mm, die Wandstärke des Röhrchens 17 etwa 0,1 mm. Die Folie 21 wird entsprechend der Form des Röhrchens gewölbt oder flach über die Öffnung gespannt und mit diesem beispielsweise durch einen Laserschweißer gasdicht verschweißt.

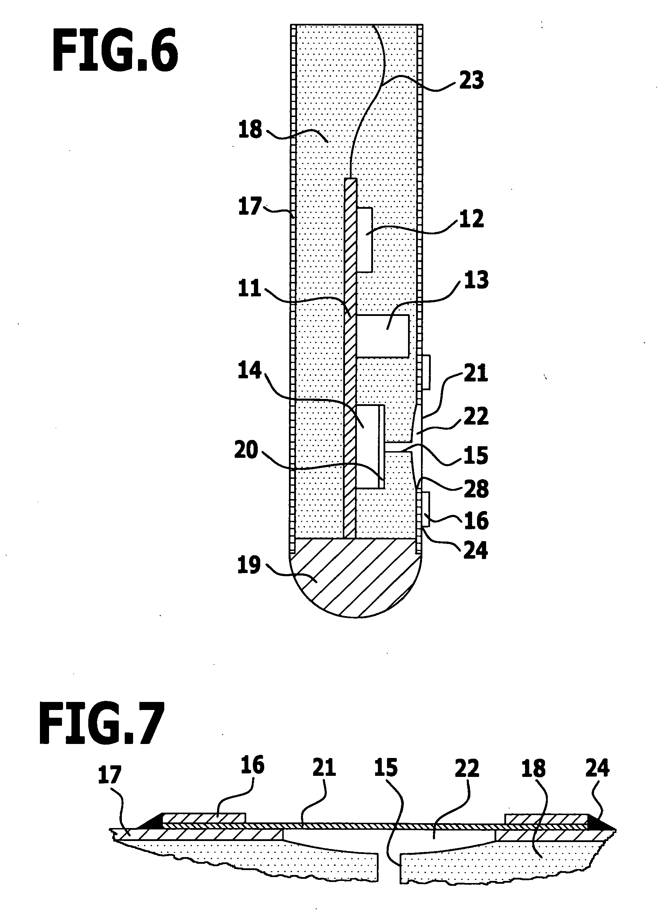

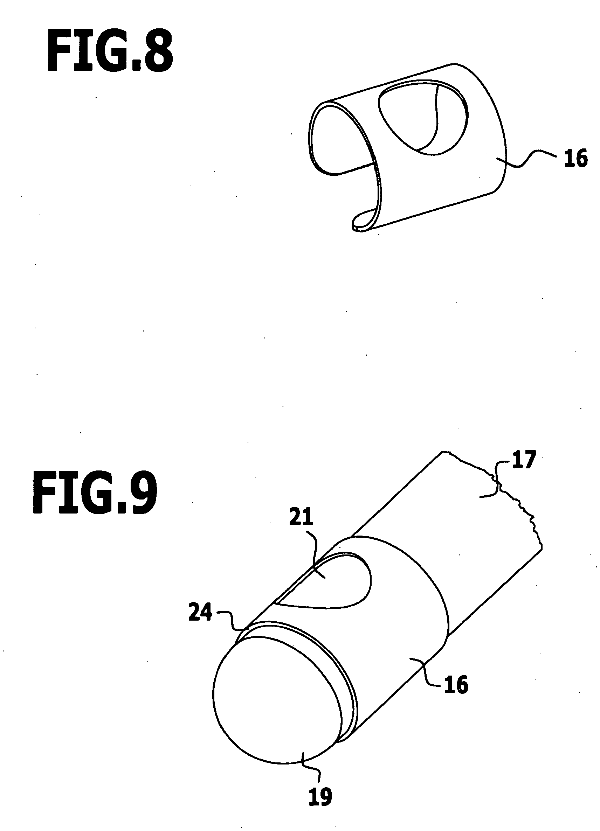

Das Verschweißen kann vorzugsweise mit Hilfe einer Klemmhülse 16 erfolgen. Figur 8 zeigt den Aufbau einer solchen Klemmhülse. Figur 6 zeigt den Aufbau eines Drucksensors mit Klemmhülse 16, Mikrochip 14, Elektronik 12, 13 und Luftkammern 15, 20, 22. Die Klemmhülse 16 hat einen Innendurchmesser, der dem Außendurchmesser des Röhrchen 17 (also des Gehäuses) entspricht. Mit Hilfe der Klemmhülse 16 kann die dünne Folie 21 über dem Fenster im Röhrchen 17 platziert und fixiert werden. Die Klemmhülse 16 weist ein ebensolches Fenster auf wie das Röhrchen 17. Die Klemmhülse 16 wird so über dem Fenster platziert, dass die beiden Fenster genau übereinander liegen. wobei das Fenster des Röhrchens 17 durch die Titanfolie bedeckt wird. Durch Verschweißen der Klemmhülse 16 mit dem Röhrchen 17 entlang der äußeren Kante 24 gelingt ein gasdichtes Verschweißen von Folie 21, Röhrchen 17 und Klemmhülse 16. Die Qualitätssicherung erfolgt mit Hilfe eines Helium-Lecksuchgerätes.

Endständig wird das Röhrchen 17 mit einer Kappe 19 verschlossen und verschweißt. Die Elektronikbauteile werden auf einem Träger 11 platziert, durch eine Kabelverbindung 23 zu einer Spule 29 wird das Senden des Messsignals sichergestellt.

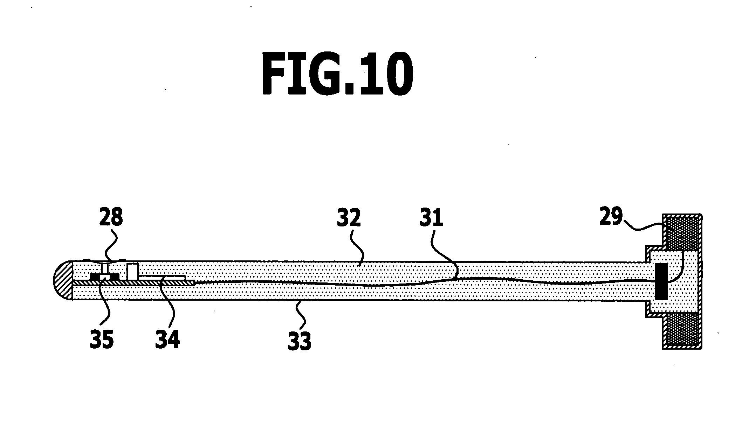

Figur 10 zeigt eine Übersichtsdarstellung der implantierbaren Vorrichtung. Über das von der Folie 21 verschlossene Fenster 28 wird der extern anliegende Druck auf die Innenkammer 35 übertragen und über die Elektronik 34 gemessen. Ein Kabel 31 leitet das Signal an die Spule 29 weiter. Durch eine geeignete Form des Gehäuses 33 wird eine passgenaue Platzierung des Gehäuses 33 in einem Schädelbohrloch möglich. Das Gehäuse 33 wird mit einem Füllstoff (vorzugsweise aus Kunststoff, Keramik oder Metall) 32 möglichst so gefüllt, dass das mit Gas gefüllte Volumen der Innenkammer 35 minimal klein ausfällt, wodurch eine möglichst sensible Druckübertragung über das Fenster 28 sichergestellt wird.

Figur 7 zeigt den exemplarischen Aufbau eines Druckfensters. Die Membrane oder Folie 21 wird über die Schweißnaht der äußeren Kante 24 mit der Klemmhülse 16 und dem Röhrchen 17 gasdicht verschlossen. Unterhalb der Folie 21 befindet sich eine minimal klein ausgelegte Luftkammer 22, die durch eine kanalförmige Luftkammer 15 mit einer Kammer 20 (Figur 6) verbunden wird. Das Füllmaterial 18 stellt ein minimal kleines Luftvolumen in den Kammern 20, 22 und der Luftkammer 15 sicher.

Figur 9 zeigt eine Aufsicht des Ventrikelsensors mit der Kappe 19, der verschweißten äußeren Kante 24, der Klemmhülse 16, dem Fenster mit der dünnen Folie 21 sowie dem Röhrchen 17. Figur 8 zeigt ein Ausführungsbeispiel für eine Klemmhülse 16.

Der Mikrochip kann, wie dies in Figur 5 dargestellt ist, ein integrierter Chip sein, der mehrere Funktionsbereiche aufweist. Ein Funktionsbereich kann beispielsweise ein druckempfindlicher Sensor 41 sein, daneben sind andere Sensoren 42 ,43 ,44 angedeutet, beispielsweise kann es sich bei einem dieser Sensoren um einen Temperatursensor handeln.

Weiterhin weist der Mikrochip einen Analog-Digital-Wandler 45 auf, in dem die von den Sensoren erzeugten analogen elektrischen Signale in Digitalsignale umgewandelt werden.

In dem dargestellten Ausführungsbeispiel ist weiterhin eine digitale Ablaufsteuerung 46 vorgesehen sowie ein Kennzeichenfeld 47, in dem eine nicht änderbare Kennzeichnung des Mikrochips 4 gespeichert sein kann, die auslesbar ist und durch die der Mikrochip 4 und damit die gesamte implantierbare Vorrichtung identifizierbar sind.

Schließlich können Signalübertragungsmittel 48 in den Mikrochip 4 integriert sein.

Bei dem Ausführungsbeispiel der Figur 5a ist im Gehäuse 1 zusätzlich ein Träger 37 in Form einer biegesteifen, dünnen Platine dargestellt, auf den der Mikrochip 4 aufgesetzt ist, beispielsweise aufgeklebt. Zusätzlich zu dem Mikrochip 4 trägt der Träger 37 weitere elektronische Bauelemente 38, beispielsweise Dioden oder Kondensatoren zur Leistungsbegrenzung, wobei es sich insbesondere um passive elektronische Bauelemente handelt. Außerdem sind auf dem Träger 37 Leiterbahnen 39 und bandförmige Kontakte 40 angeordnet, die einerseits den Mikrochip 4 und die Bauelemente 38 und andererseits die Bauelemente 38 untereinander verbinden.

Bei allen Ausführungsformen ist es möglich, entweder im Gehäuse 1 nur einen Mikrochip anzuordnen, wie dies aus der Darstellung der Figur 5 ersichtlich ist, oder aber einen Träger 37, auf dem neben dem Mikrochip 4 noch weitere Bauelemente 38 sowie Leiterbahnen 39 und Kontakte 40 angeordnet sind.

Bei allen nachfolgend dargestellten Ausführungsbeispielen kann zusätzlich dieser Träger zu dem Mikrochip hinzugefügt werden, in den Zeichnungen ist dies jedoch nur beim Ausführungsbeispiel der Figur 5a dargestellt.

Der Mikrochip 4 kann in dem Röhrchen 17 so angeordnet sein, wie es anhand der Figur 6 erläutert worden ist.

Es kann aber auch vorgesehen sein, dass das Gehäuse 1 so klein ausgebildet ist, dass es gerade ausreicht, den Mikrochip 4 aufzunehmen, wie dies am Beispiel der Figur 5 schematisch dargestellt ist. In diesem Falle füllt der Mikrochip 4 fast den gesamten Innenraum des Gehäuses 1 aus, der verbleibende Innenraum ist mit dem Übertragungsmittel 5 ausgefüllt, insbesondere mit einem hochviskosen Öl. Die Sensoren 41 bis 44 und insbesondere der druckempfindliche Sensor 41 befinden sich etwa in der Mitte des Gehäuses 1, wie dies ebenfalls in Figur 5 dargestellt ist. Das Gehäuse 1 kann zylindrisch ausgebildet sein mit einem Außendurchmesser in der Größenordnung zwischen 2 und 5 Millimetern, insbesondere bei etwa 3 Millimetern, und einer Länge zwischen 15 und 25 Millimetern, insbesondere von etwa 20 Millimetern. Es handelt sich somit um eine sehr kleine Baueinheit, die in einfacher Weise an der gewünschten Stelle im Gehirn platziert werden kann.

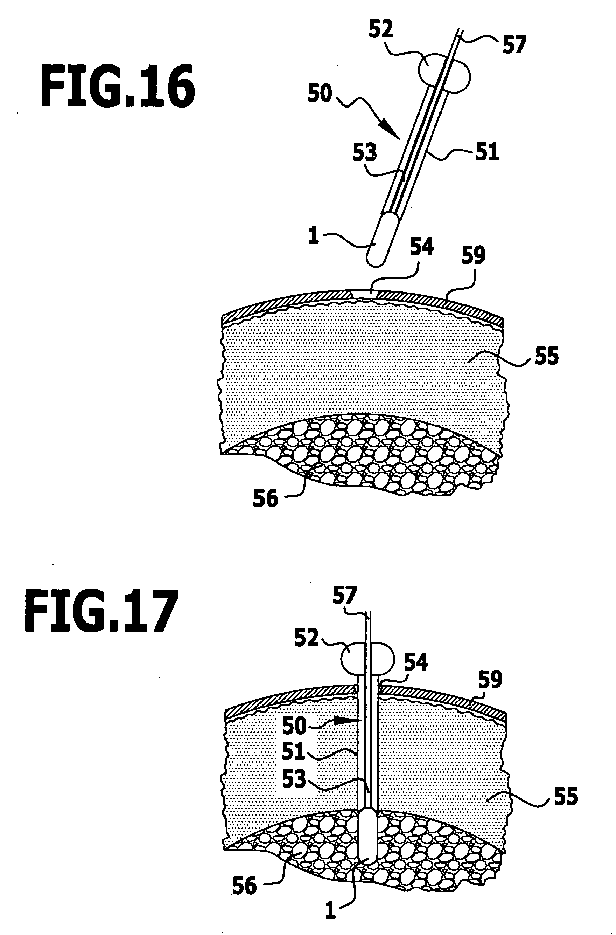

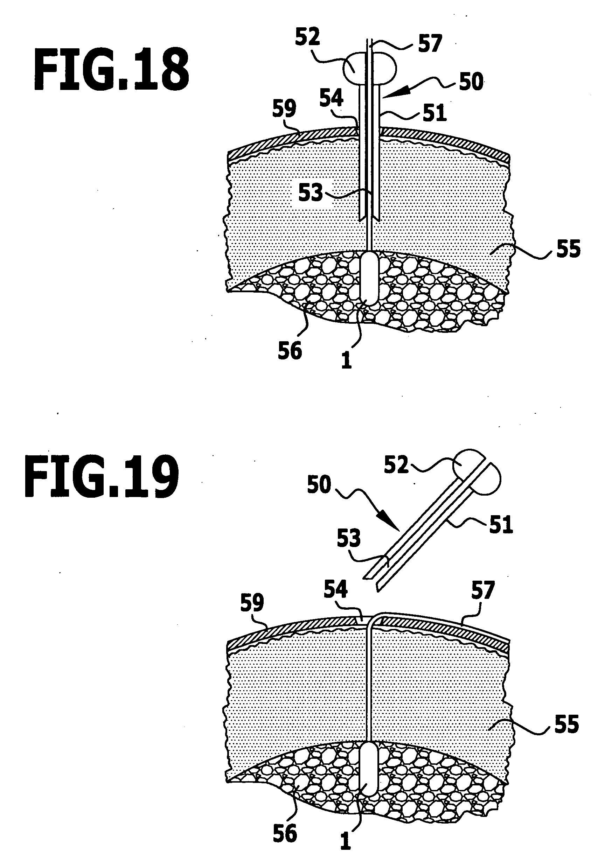

Diese Platzierung kann mit Hilfe eines Handhabungsinstrumentes 50 erfolgen, wie es in den Figuren 16 bis 19 schematisch dargestellt ist. Es handelt sich dabei um ein Röhrchen 51 mit einem Handgriff 52, dessen Außenwand einen durchgehenden Längsschlitz 53 aufweist. Das Gehäuse 1 wird am vorderen Ende des Handhabungsinstrumentes 50 in das Röhrchen 51 eingeschoben und dort, beispielsweise durch Klemmung, gehalten. Das Handhabungsinstrument 50 wird mit dem darin gehaltenen Gehäuse 1 durch ein Bohrloch 54 in der Schädeldecke 55 an der gewünschten Stelle des Gehirnes 56 platziert (Figur 17), und anschließend wird das Handhabungsinstrument 50 wieder aus dem Bohrloch 54 zurückgezogen, wobei das Gehäuse 1 im Gehirn 56 verbleibt (Figur 18). Der Längsschlitz 53 dient dabei dazu, ein am Gehäuse 1 angeordnetes Verbindungskabel 57 in das Röhrchen 51 einzulegen und nach der Platzierung des Gehäuses 1 wieder aus diesem herauszuziehen, so dass das Handhabungsinstrument 50 nach der Platzierung des Gehäuses 1 von diesem vollständig getrennt werden kann (Figur 19).

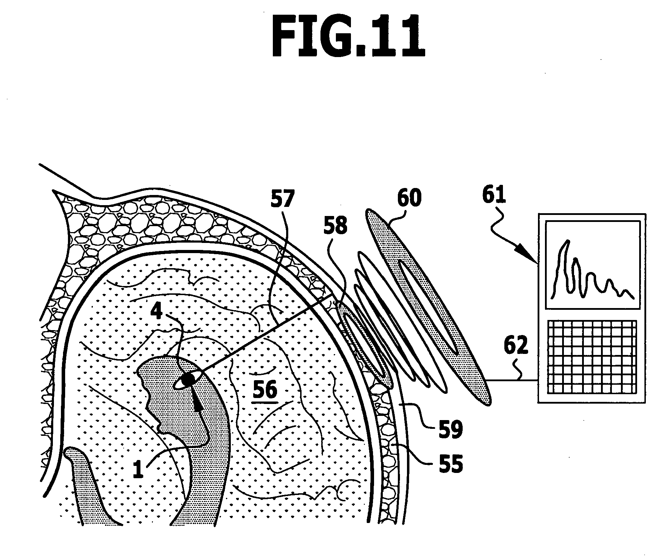

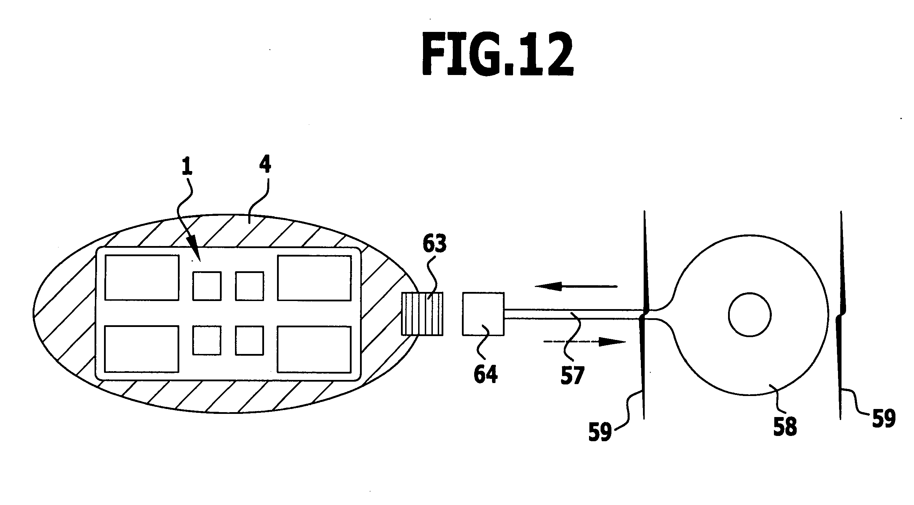

Figur 11 zeigt das in dieser Weise im Gehirn 56 platzierte Gehäuse 1 und ein von dem Gehäuse 1 auf die Außenseite der Schädeldecke 55 führendes Verbindungskabel 57, welches mit einer Spule 58 verbunden ist, die außen auf die Schädeldecke 55 aufgelegt ist, und zwar zwischen Schädeldecke 55 und Kopfhaut 59 oder bei einer alternativen Ausführungsform außen auf die Kopfhaut 59. In Figur 12 sind beide Alternativen dadurch dargestellt, dass die Kopfhaut 59 zweimal dargestellt ist, nämlich einmal auf der einen Seite und einmal auf der anderen Seite der Spule 58.

Diese Spule 58 kann mit einer Übertragungsspule 60, die außen an die Kopfhaut 59 herangeführt wird, induktiv gekoppelt werden, so dass über die beiden Spulen 58 und 60 eine elektrische Verbindung zu einer Auswerteeinheit 61 hergestellt werden kann, die über eine Leitung 62 mit der Übertragungsspule 60 verbunden ist.

In ähnlicher Weise wird die Spule 29 bei dem Ausführungsbeispiel der Figuren 6 bis 10 mit einer Auswerteeinheit verbunden.

Diese Verbindung kann aber auch ersetzt werden durch eine galvanische Verbindung, bei welcher das vom Gehäuse 1 kommende Verbindungskabel 57 nicht mit einer Spule 58 verbunden ist, sondern direkt mit einer extrakorporalen Auswerteeinheit, die beispielsweise am Körper getragen wird. In diesem Falle durchsetzt das Verbindungskabel 57 die Kopfhaut.

Bei dem Ausführungsbeispiel der Figuren 6 bis 10 ist die Spule 29 in das Gehäuse 33 eingebettet, so dass eine elektrisch leitende Verbindung zwischen dem Mikrochip 4 und der Spule 29 im Inneren des Gehäuses erfolgen kann.

Anders ist dies bei den Ausgestaltungen gemäß Figuren 5 oder 5a, bei denen in dem Gehäuse 1 nur der Mikrochip 4 beziehungsweise der Träger 37 mit Mikrochip 4 angeordnet sind, hier ist ein Verbindungskabel 57 notwendig, welches aus dem Gehäuse 1 herausgeführt werden muss. Diese Durchführung ist so ausgebildet, dass das Innere des Gehäuses 1 in diesem Austrittsbereich hermetisch abgedichtet ist. Dies kann beispielsweise mittels eines in die Gehäusewand eingesetzten und gegenüber dieser abgedichteten Trägers aus Keramik oder Kunststoff erfolgen, in den elektrische Kontakte eingelassen sind. Zur Abdichtung kann ein Kleber oder ein Goldlot verwendet werden.

An die Kontakte der hermetischen Durchführung kann das Verbindungskabel 57 dauerhaft angeschlossen werden, beispielsweise durch Löten, Schweißen, Kontaktkleben, Crimpen oder andere an sich bekannte Verbindungstechniken.

Bei einer anderen Ausgestaltung kann auch eine lösbare Verbindung zwischen der hermetischen Durchführung und dem Verbindungskabel vorgesehen sein, beispielsweise unter Verwendung einer Steckverbindung. In dem Ausführungsbeispiel der Figur 12 ist schematisch ein solcher Steckkontakt 63 am Gehäuse 1 dargestellt, der die Wand des Gehäuses 1 abgedichtet durchsetzt und auf den das Verbindungskabel 57 mittels eines passenden Gegenstücks 64 aufgesteckt werden kann.

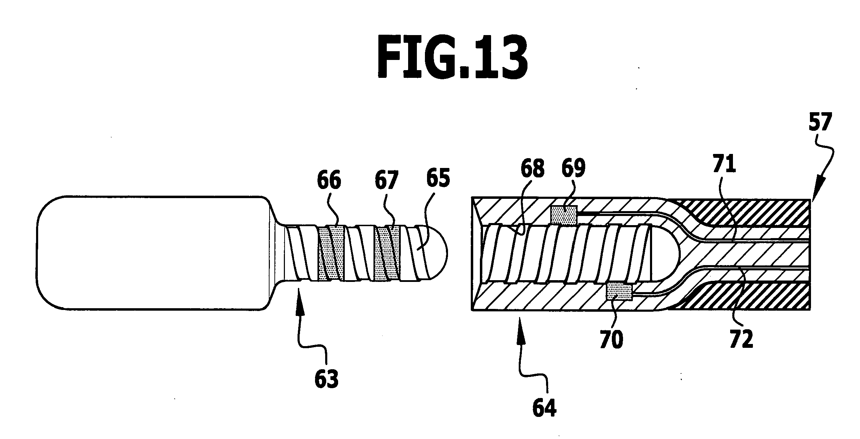

Ein Ausführungsbeispiel eines solchen Steckkontaktes 63 und eines entsprechenden Gegenstückes 64 ist in Figur 13 dargestellt.

Der am Gehäuse 1 angeordnete Steckkontakt 63 weist einen Außengewindezapfen 65 auf, der zwei elektrisch voneinander isolierte Kontaktbereiche 66, 67 trägt, diese stehen über getrennte Leitungen mit dem Mikrochip 4 in Verbindung.

Das Gegenstück 64 weist eine Innengewindebohrung 68 auf, so dass das Gegenstück auf den Außengewindezapfen 65 aufgeschraubt werden kann. Beim vollständigen Aufschrauben gelangen zwei Kontaktbereiche 69 und 70 in elektrisch leitende Anlage an den Kontaktbereichen 66 bzw. 67, so dass in diesen Kontaktbereichen eine elektrische Verbindung hergestellt wird. Die Kontaktbereiche 69 und 70 des Gegenstücks 64 sind mit Adern 71, 72 des Verbindungskabels 57 verbunden.

Nach dem Aufschrauben verschließt das Gegenstück 64 den Außengewindezapfen 65 vollständig und dichtet diesen gegenüber der Umgebung ab. Man erhält auf diese Weise nicht nur eine hermetisch dichte Durchführung durch die Wand des Gehäuses, sondern auch eine hermetisch dichte Verbindung des Steckkontaktes 63 mit dem Gegenstück 64.

Diese Verbindung kann natürlich auch als einfache Steckverbindung ausgebildet sein, daher wird der Ausdruck Steckkontakt verwendet. Günstig ist aber die beschriebene Schraubverbindung, weil dadurch ein unbeabsichtigtes Lösen der Verbindung verhindert wird.

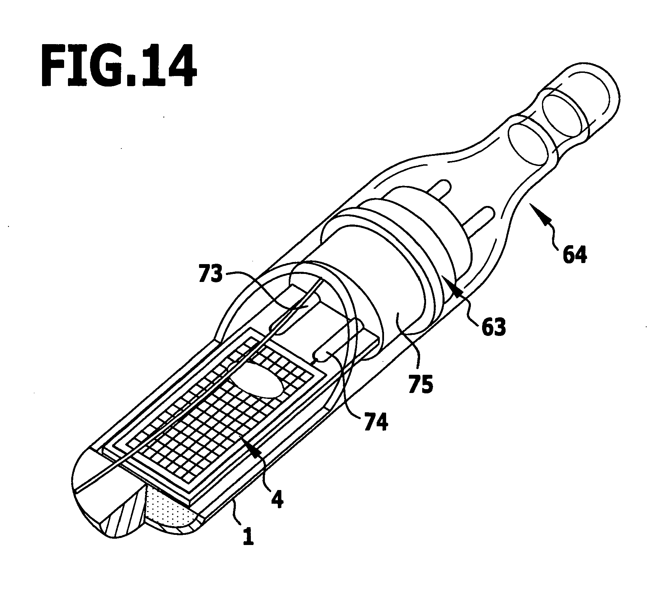

In Figur 14 ist ein Ausführungsbeispiel eines derartigen Gehäuses 1 dargestellt, welches in seinem Inneren den Mikrochip 4 aufnimmt, der unmittelbar mit Kontaktstiften 73, 74 in Verbindung steht. Diese durchsetzen dicht einen Träger 75, der dicht in die Wand des Gehäuses 1 eingesetzt ist und auf den ein Gegenstück 64 aufgesteckt ist. Die Kontaktstifte 73, 74 können auch unmittelbar mit der Verbindungsleitung dauerhaft verbunden sein, beispielsweise durch Schweißen, Löten, Kontaktkleben, Crimpen oder andere Techniken.

Das Gehäuse 1 ist in seinem verbleibenden Innenraum mit einem hochviskosen Öl gefüllt und überträgt in der beschriebenen Weise Bewegungen der in Figur 14 nicht dargestellten Membran auf die Sensoren des Mikrochips 4.

Die gesamte in Figur 14 dargestellte Anordnung weist einen Durchmesser von größenordnungsmäßig 3 Millimetern und eine Länge von größenordnungsmäßig 20 Millimetern auf, stellt also eine sehr kleine Baueinheit dar.

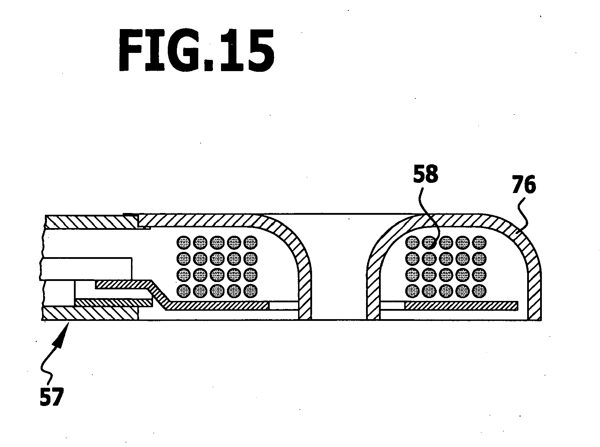

Wenn das Verbindungskabel 57 an seinem dem Gehäuse 1 abgewandten Ende mit einer Spule 58 verbunden wird, so kann dies in einer Weise erfolgen, wie dies aus der Darstellung der Figur 15 deutlich wird. In einem ringförmigen, allseits geschlossenen Gehäuse 76 wird die Spule aufgenommen, seitlich mündet in das Gehäuse 76 abgedichtet das Verbindungskabel 57 ein und ist dort mit der Spule 58 verbunden. Es ergibt sich eine sehr flache Anordnung, die in dieser Form auf den Schädelknochen aufgelegt werden kann, und zwar zwischen Schädelknochen und Kopfhaut, wie dies aus der Darstellung der Figur 11 deutlich wird.

Über die Spule 58 und das Verbindungskabel 57 kann einerseits Energie von außen dem Mikrochip 4 zugeführt werden, so dass dieser keine eigene Energiequelle benötigt. Andererseits können von den Sensoren des Mikrochips 4 erzeugte Digitalsignale über die Verbindungsleitung zu der Auswerteeinheit übertragen werden. Es handelt sich damit bei dem Verbindungskabel 57 um eine Energie- und Signalübertragungsleitung.

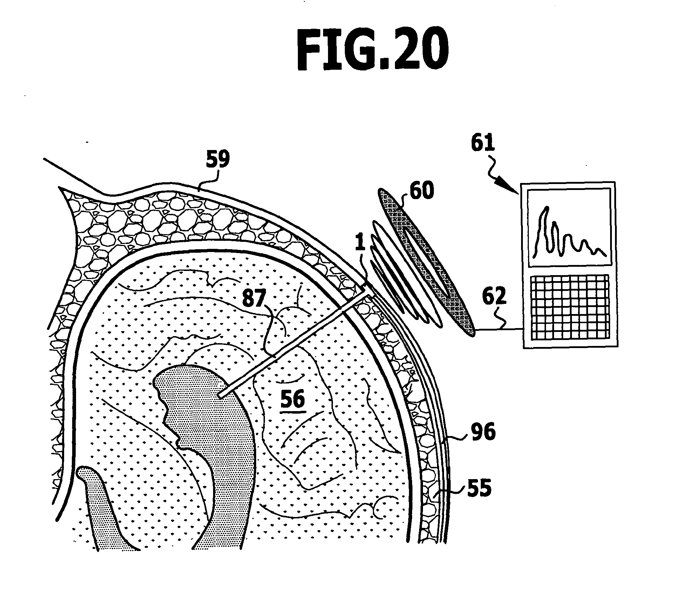

Eine weitere bevorzugte Ausführungsform einer implantierbaren Vorrichtung zur intracraniellen Druckmessung ist in den Figuren 20 ff dargestellt. Das Gehäuse 1 dieser Vorrichtung ist in diesem Falle in Form einer flachen Dose ausgebildet mit einer ebenen Bodenfläche 80 und einer im dargestellten Ausführungsbeispiel ebenfalls ebenen Oberseite 81. Das Gehäuse ist dabei im Querschnitt kreisförmig geformt mit einem Durchmesser zwischen 1 cm und 3 cm, die Höhe beträgt etwa zwischen 2 mm und 5 mm. Das Gehäuse 1 ist durch eine parallel zur Bodenfläche 80 verlaufende Zwischenwand 82 in eine obere Messkammer 83 und in eine untere Flüssigkeitskammer 84 unterteilt. Im mittleren Bereich ist die Zwischenwand 82 durchbrochen, und dieser Verbindungsbereich 85 zwischen der Messkammer 83 und der Flüssigkeitskammer 84 wird durch die Membran 3 verschlossen.

Am unteren Ende der Flüssigkeitskammer 84 tritt in der Mitte des Gehäuses 1 ein Rohrstutzen 86 senkrecht nach unten abstehend aus, dieser ist verbunden mit einem länglichen Röhrchen 87, welches einen Katheter ausbildet.

In der Messkammer 83 ist ebenso wie in den Gehäusen der oben beschriebenen Ausführungsformen ein Mikrochip 4 angeordnet, die Messkammer 83 ist mit dem Übertragungsmittel 5 ausgefüllt. Auf diese Weise kann über die Membran 3 der Druck einer Flüssigkeit in der Flüssigkeitskammer 84 gemessen und ein entsprechendes Messsignal erzeugt werden.

Die beschriebene Vorrichtung wird so am Kopf platziert, dass das Röhrchen 87 nach Art eines Katheters mit seinem freien Ende an den Ort des Gehirns vorgeschoben wird, an dem der intracranielle Druck gemessen werden soll. Das Gehäuse 1 liegt mit seiner Bodenfläche 80 auf der Außenseite der Schädeldecke 55 auf, der Rohrstutzen 86 und das Röhrchen 87 ragen dabei durch das Bohrloch 54 in der Schädeldecke 55 hindurch, wie dies in Figur 20 schematisch dargestellt ist. Das Gehäuse 1 dient damit als Bohrlochabdeckung. Das Röhrchen 87 ist an seinem dem Gehäuse 1 abgewandten Ende offen und ermöglicht somit der Hirnflüssigkeit, in die Flüssigkeitskammer 84 einzuströmen. Bei vollständiger Füllung wird der Druck der Hirnflüssigkeit am Eintrittsort über die Membran 3 auf den Mikrochip 4 in der Messkammer 83 übertragen.

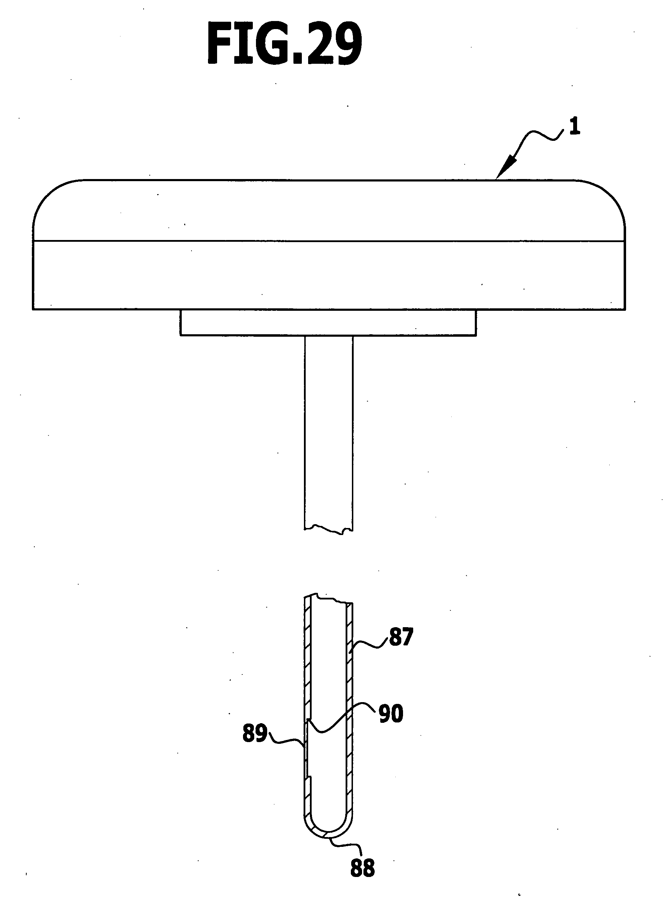

Es könnte aber auch, wie in Figur 29 dargestellt, das Röhrchen 87 verschlossen sein und an seinem verschlossenen Ende 88 eine von einer Membran 89 verschlossene Öffnung 90 aufweisen. Bei dieser Ausführungsform sind die Flüssigkeitskammer 84 und das Röhrchen 87 mit einem weiteren Übertragungsmedium angefüllt, beispielsweise einer Flüssigkeit, und der Druck der umgebenden Hirnflüssigkeit überträgt sich über die Membran 89 auf die Flüssigkeitsfüllung in der Flüssigkeitskammer und im Röhrchen 87. Auf diese Weise wird der Druck durch das Übertragungsmedium auf die Membran 3 und damit auf den Mikrochip 4 übertragen.

Die vom Mikrochip erzeugten digitalen Signale werden entweder über eine in der Messkammer 83 angeordnete Spule 91 auf eine Übertragungsspule 60 übertragen oder mittels eines abgedichtet aus dem Gehäuse 1 heraus geführten Verbindungskabels 57 galvanisch oder induktiv an die Auswerteeinheit weitergeleitet.

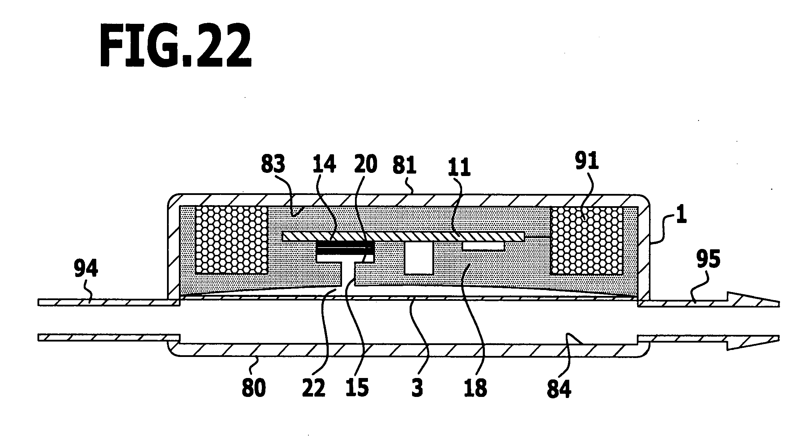

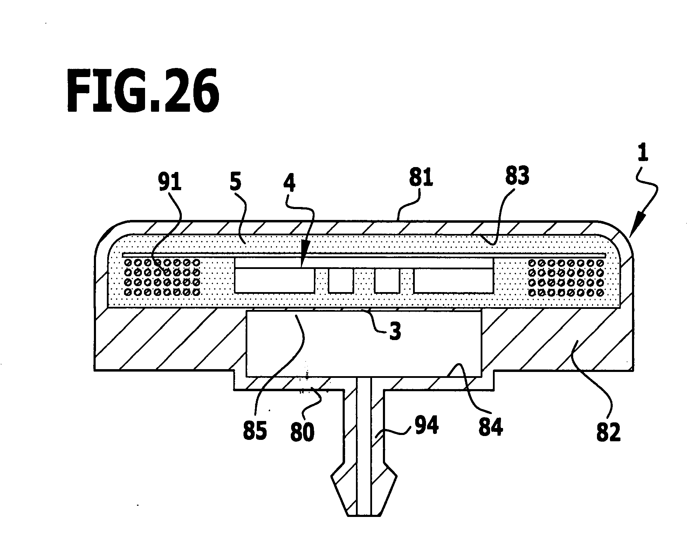

Bei den Ausführungsbeispielen der Figuren 22, 23, 25 und 26 umgibt die Spule 91 den Mikrochip im Innern der Messkammer 83 konzentrisch, so dass eine besonders günstige Raumaufteilung erreicht wird, die zu einer kleinen Baugröße des Gehäuses 1 beiträgt.

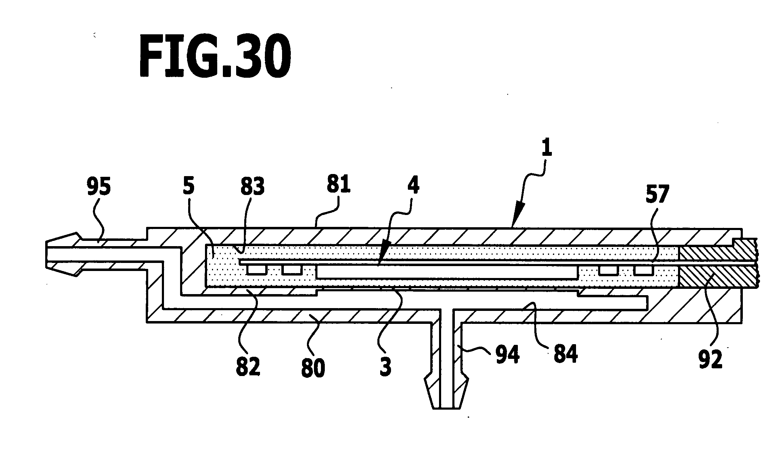

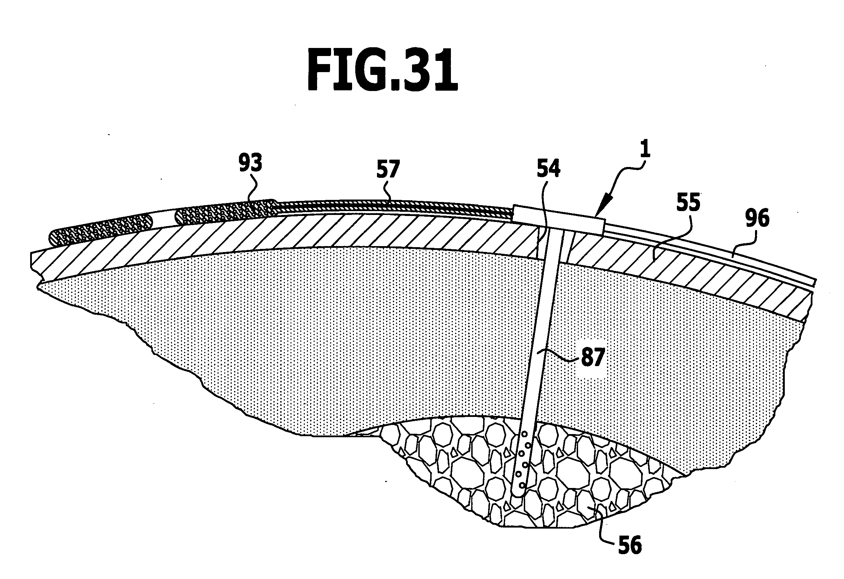

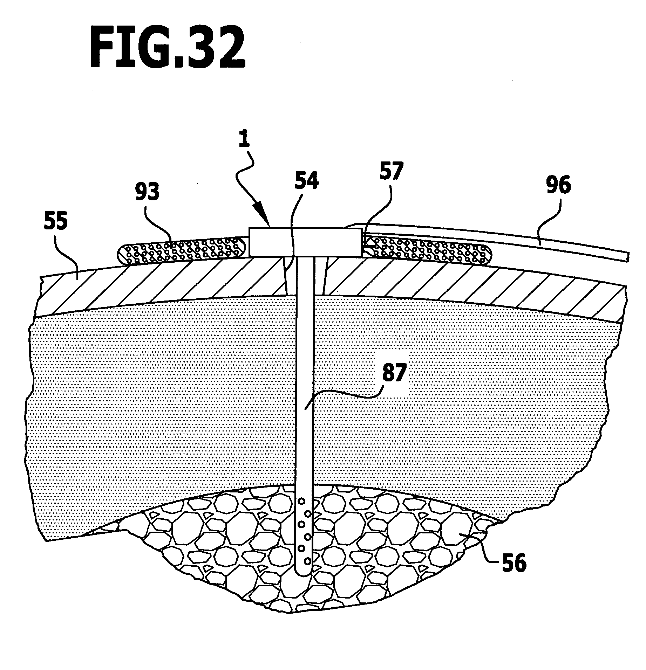

Bei den Ausführungsbeispielen der Figuren 30, 31 und 32 dagegen ist ein aus dem Gehäuse 1 heraus führendes Verbindungskabel 57 vorgesehen. Im Austrittsbereich ist das Gehäuse 1 dabei mit einer abgedichteten Kabeldurchführung 92 versehen, die auch so ausgebildet sein kann, wie es anhand der übrigen Ausführungsbeispiele erörtert worden ist. Das Verbindungskabel kann unmittelbar zu einer Spule 93 führen, die außerhalb des Gehäuses 1 auf die Außenseite der Schädeldecke 5 aufgelegt ist, und zwar entweder im Abstand von dem Gehäuse 1 neben diesem (Figur 31) oder das Gehäuse 1 konzentrisch umgebend (Figur 32).

Natürlich könnte das Verbindungskabel 57 auch unmittelbar galvanisch mit der Auswerteeinheit 61 verbunden werden.

Die beschriebene Konstruktion eignet sich insbesondere für die Erfassung des Flüssigkeitsdruckes in einem Ableitungssystem zur Behandlung des Hydrocephalus. Figur 22 zeigt eine Übersichtsdarstellung eines derartigen Aufbaus. Bei dieser Vorrichtung, deren Messkammer ähnlich aufgebaut ist wie im Ausführungsbeispiel der Figur 6, die aber zusätzlich eine Spule 91 aufnimmt, ist die Zwischenwand 82 ersetzt durch die Membran 3, d.h. die Membran 3 erstreckt sich über den gesamten Querschnitt des Gehäuses 1 und unterteilt den Innenraum des Gehäuses 1 in die Messkammer 83 und in die Flüssigkeitskammer 84. Eine solche Ausbildung kann auch bei den anderen dosenförmigen Gehäusen 1 Verwendung finden, es kann aber auch bei allen derartigen dosenförmigen Gehäusen eine Ausbildung mit Zwischenwand 82 und einer in diese eingesetzten Membran 3 Verwendung finden.

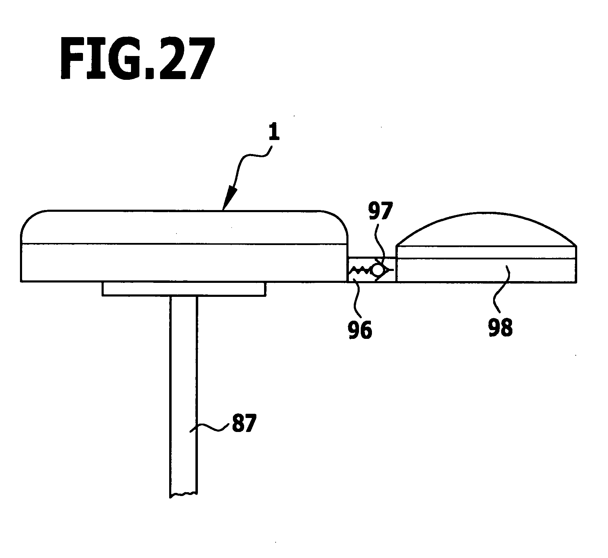

Von der Flüssigkeitskammer 84 zweigen bei dem Ausführungsbeispiel der Figur 22 auf gegenüberliegenden Seiten zwei parallel zur Bodenfläche 80 verlaufende Rohrstutzen 94, 95 ab, von denen einer mit einem Röhrchen 87 verbunden ist und den Flüssigkeitszulauf ausbildet, während der andere mit einer Flüssigkeitsableitung verbunden werden kann, die schematisch lediglich in den Figuren 31 und 32 erkennbar ist. Damit kann Hirndruckflüssigkeit aus dem Schädelinneren durch die Flüssigkeitskammer 84 hindurchströmen und aus dem Schädelinnenraum entfernt werden, wie dies bei Ableitungssystem zur Behandlung des Hydrocephalus üblich ist. Wie in Figur 27 schematisch dargestellt ist, kann in eine Ablaufleitung 96 ein Rückschlagventil 97 eingesetzt sein, die Ablaufleitung kann in einem Reservoir 98 enden, in dem die abgeführte Flüssigkeit gesammelt wird. Dieser Vorrat kann z.B. verwendet werden, um die Flüssigkeitswege rückzuspülen und zu reinigen.

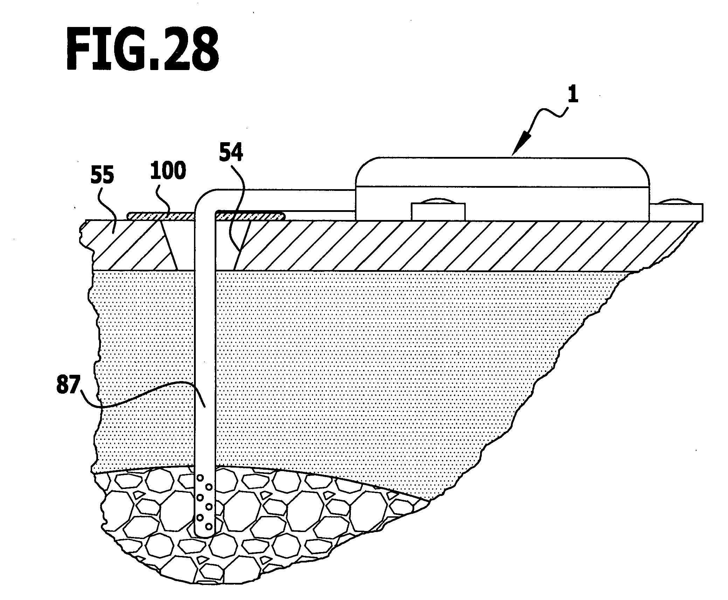

Bei einem Gehäuse 1 mit einer parallel zur Bodenfläche 80 in die Flüssigkeitskammer 84 eintretender Zulaufleitung ist es günstig, das Gehäuse 1 nicht unmittelbar über einem Bohrloch 54 anzuordnen, sondern seitlich neben einem Bohrloch, so dass das Bohrloch für den Durchtritt der Zulaufleitung freibleibt, wie dies in Figur 28 dargestellt ist. Es kann dann im Bereich des Bohrlochs 54 eine separate Bohrlochabdeckung 100 vorgesehen werden.

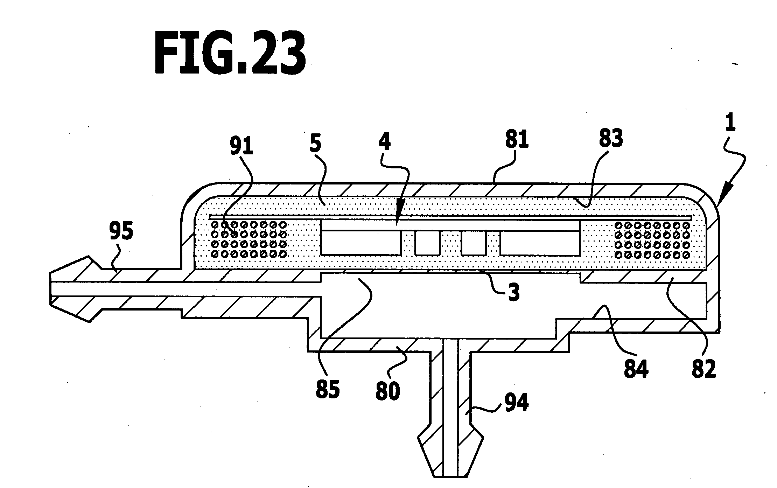

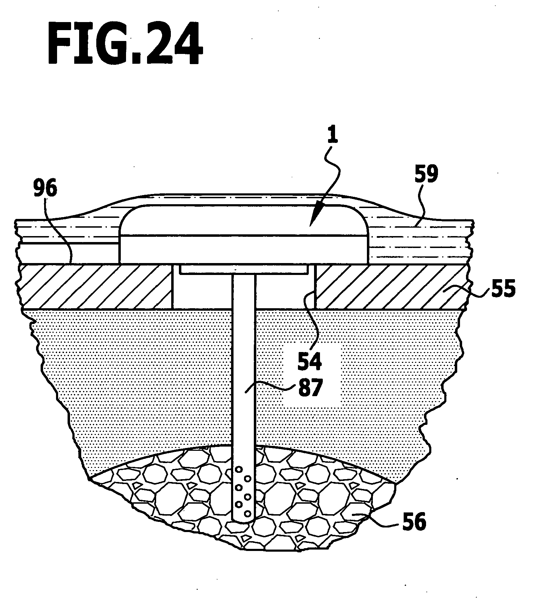

Bei dem Ausführungsbeispiel der Figur 22 verlaufen beide Rohrstutzen 94 und 95 parallel zur Bodenfläche, es sind auch Anordnungen möglich, wie sie in Figur 23 dargestellt sind. Dabei mündet ein Rohrstutzen 94 von unten her senkrecht zur Bodenfläche 80 zentral in die Flüssigkeitskammer 84 ein, während der zweite Rohrstutzen 95 parallel zur Bodenfläche seitlich austritt. Eine solche Vorrichtung wird in der in Figur 24 beschriebenen Art eingesetzt, so dass das Röhrchen 87 durch das Bohrloch 54 in der Schädeldecke 55 in das Schädelinnere hineinragt, das Gehäuse 1 deckt dabei das Bohrloch 54 ab. Das Gehäuse 1 wird zwischen Schädeldecke 55 und Kopfhaut 59 angeordnet, eine Ablaufleitung 96 kann unmittelbar auf der Schädeldecke 55 und unter der Kopfhaut 59 verlaufen.

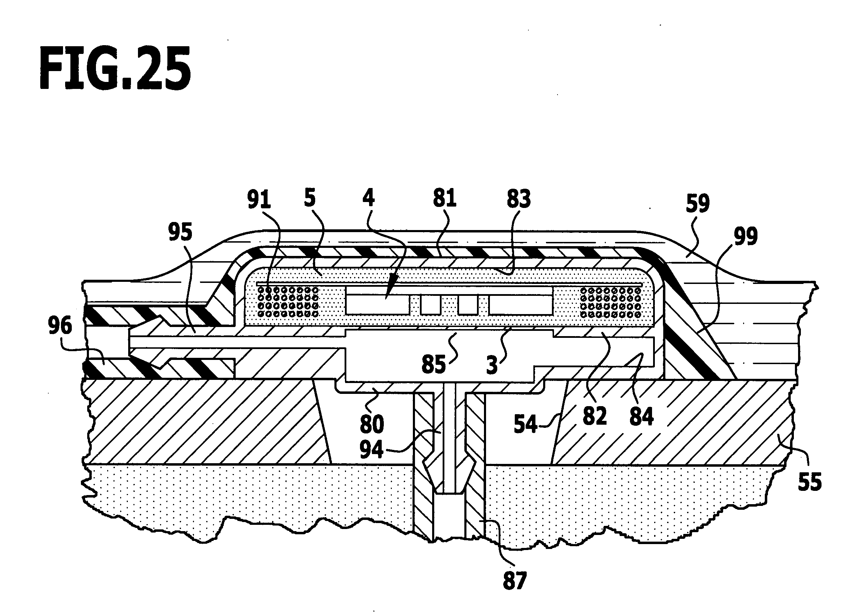

In Figur 25 ist eine ähnliche Anordnung beschrieben, dabei ist das Gehäuse 1 zusätzlich von einer Schutzkappe 99 überfangen, so dass sowohl das Gehäuse 1 als auch das umliegende Gewebe zusätzlich geschützt werden.

Die folgenden Ausführungsbeispiele fallen nicht unter die Erfindung.

Wenn als Übertragungsmittel im Inneren des Gehäuses 1 ein vulkanisiertes oder polymerisiertes Material verwendet wird, insbesondere ein vernetztes Silikon, dann kann der gesamte Zwischenraum zwischen dem Mikrochip 4 und der Membran 3 mit diesem Material ausgefüllt werden, so dass dann vollflächig eine Druckübertragung erfolgt.

Es ist aber auch möglich, dass die Druckübertragung nur in einem Teilbereich des Mikrochips erfolgt.

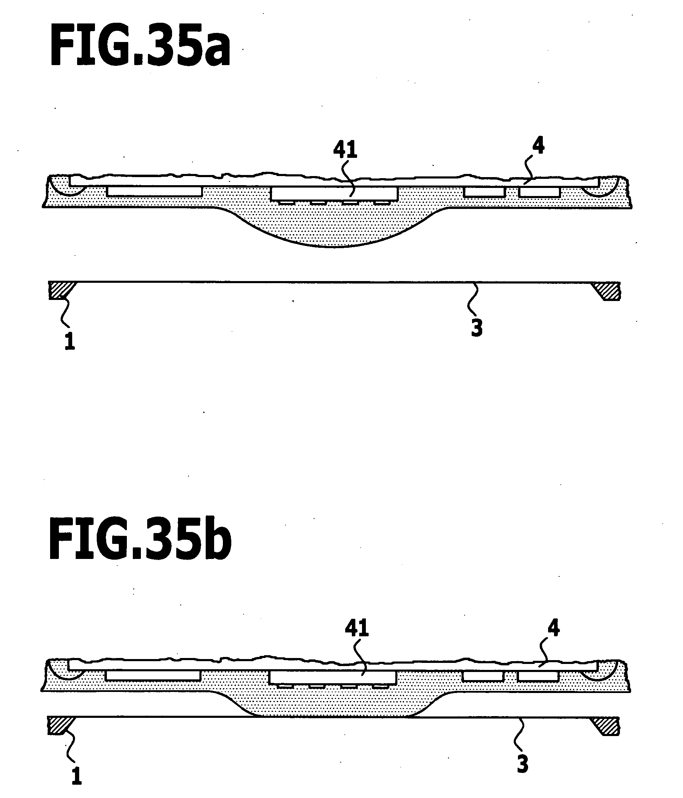

Im Ausführungsbeispiel der Figur 35a ist dargestellt, dass der Mikrochip vollflächig von einem solchen vulkanisierten oder polymerisierten Material, nachstehend kurz Übertragungsmaterial genannt, bedeckt ist, dass aber im Bereich des Drucksensors 41 dieses Übertragungsmaterial eine größere Dicke aufweist. Im eingebauten Zustand liegen der Mikrochip 4 und die Membran 3 so dicht aneinander, dass in diesem zentralen Bereich, in dem das Übertragungsmaterial eine größere Dicke aufweist, dieses an der Membran 3 anliegt, wie dies in Figur 35b dargestellt ist, so dass in diesem Bereich eine Druckübertragung stattfindet. Diese Druckübertragung ist somit auf den Bereich des Drucksensors 41 konzentriert.

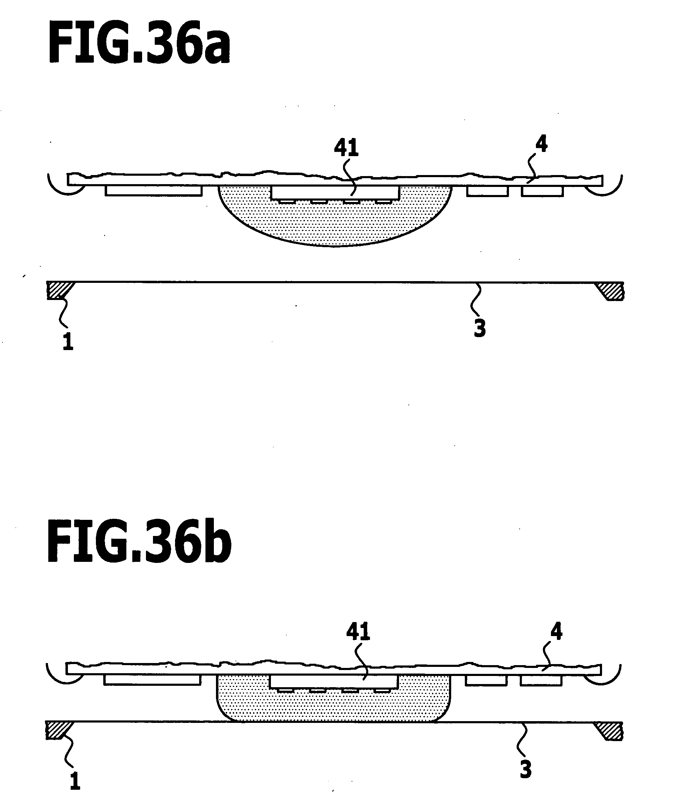

Es ist auch möglich, dass gemäß der Ausgestaltung der Figuren 36a und 36b nur der Bereich des Drucksensors 41 von dem Übertragungsmaterial umhüllt wird, während außen liegende Randbereiche des Mikrochips frei von dem Übertragungsmaterial bleiben.

Schließlich ist es möglich, dass der Mikrochip gar nicht mit dem Übertragungsmaterial beschichtet ist, sondern das Übertragungsmaterial auf der Membran 3 angeordnet ist, so dass dadurch zwischen Membran 3 und Drucksensor 41 eine druckübertragende Schicht aus dem Übertragungsmaterial gebildet wird.

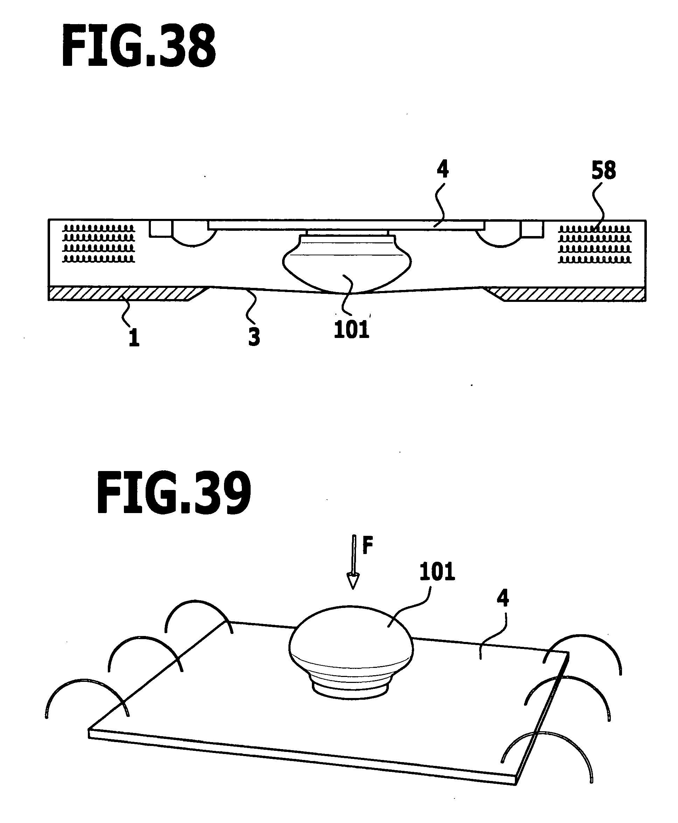

Schließlich ist es bei einem abgewandelten Ausführungsbeispiel auch möglich, die Druckübertragung durch mechanische Druckübertragungselemente vorzunehmen, beispielsweise durch einen Druckstempel 101, der am Mikrochip 4 gelagert ist und sich an der Membran 3 abstützt, wie dies in den Figuren 38 und 39 dargestellt ist. Dieser Druckstempel 101 überträgt dann die Druckkräfte von der Membran 3 auf den Drucksensor 41.

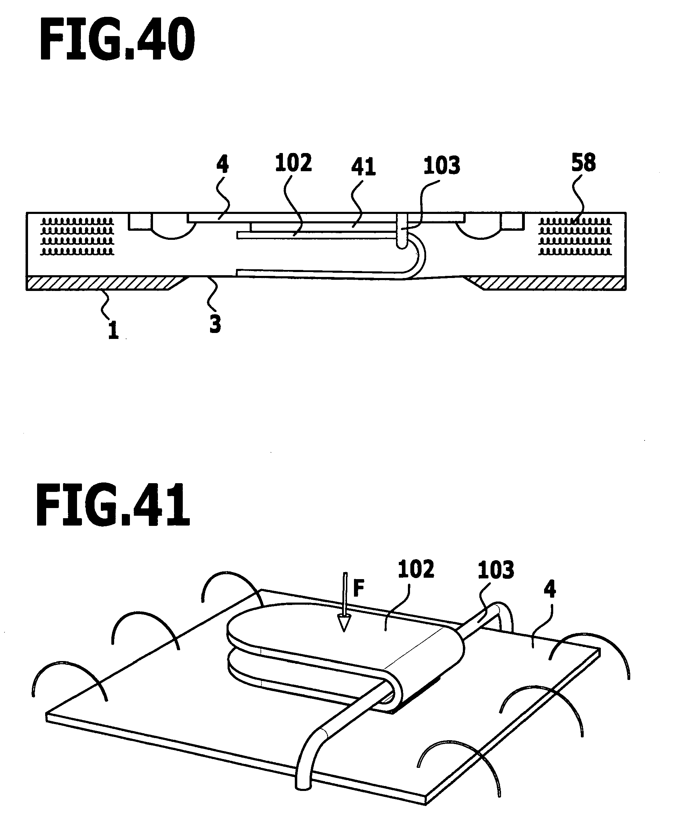

An Stelle des Druckstempels 101 kann auch zwischen Mikrochip 4 und Membran 3 ein Federelement eingesetzt werden, beispielsweise eine U-förmige Blattfeder 102, die sich einerseits an dem Drucksensor 41 und andererseits an der Membran 3 abstützt, wie dies in den Figuren 40 und 41 dargestellt ist. Eine solche Blattfeder 102 kann über seitliche Stege 103 an dem Gehäuse 1 oder an einem den Mikrochip 4 haltenden Träger 37 gelagert sein.

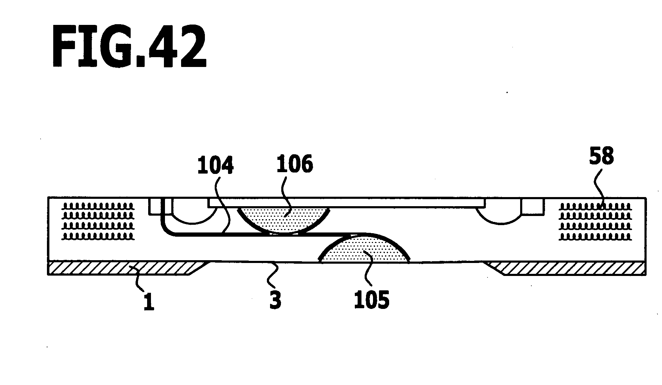

In Figur 42 ist eine abgewandelte Konstruktion für ein mechanisches Druckübertragungselement dargestellt, nämlich ein am Gehäuse 1 oder einem Träger 37 gelagerter Bügel 104, der sich mittels eines ersten Stempels 105 an der Membran 3 und mittels eines zweiten Stempels 106 am Drucksensor 41 abstützt und der auf diese Weise die Druckkräfte von der Membran 3 auf den Drucksensor 41 überträgt.

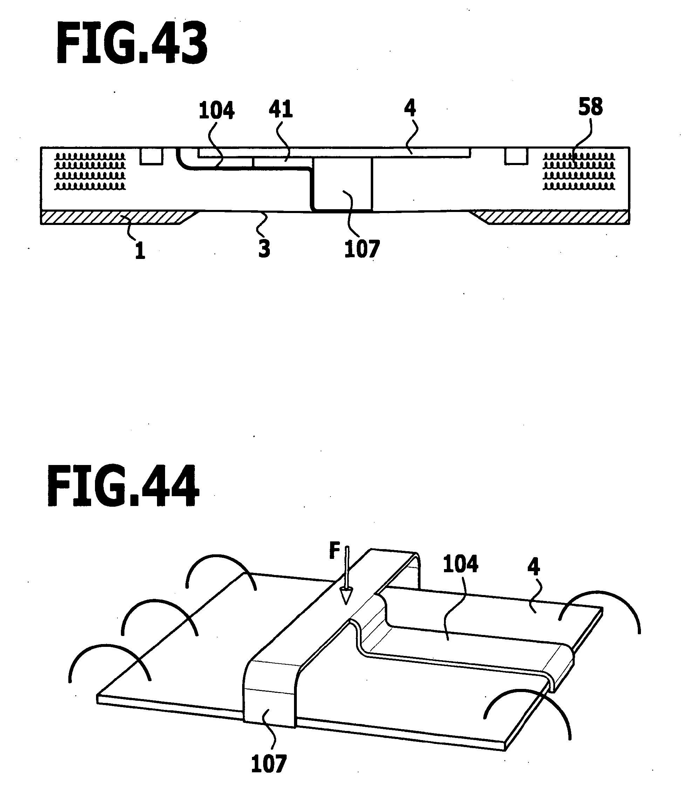

Wie aus den Figuren 43 und 44 zu entnehmen ist, kann ein solcher Bügel 104 seitliche Stege 107 tragen, die als Anschlag wirken und die Bewegung des Bügels 104 begrenzen, um dadurch eine Überlastung und Beschädigung der Gesamtanordnung zu vermeiden. Derartige Anschläge können bei allen Anordnungen vorgesehen werden, die die Druckkräfte mechanisch auf den Drucksensor 41 übertragen.

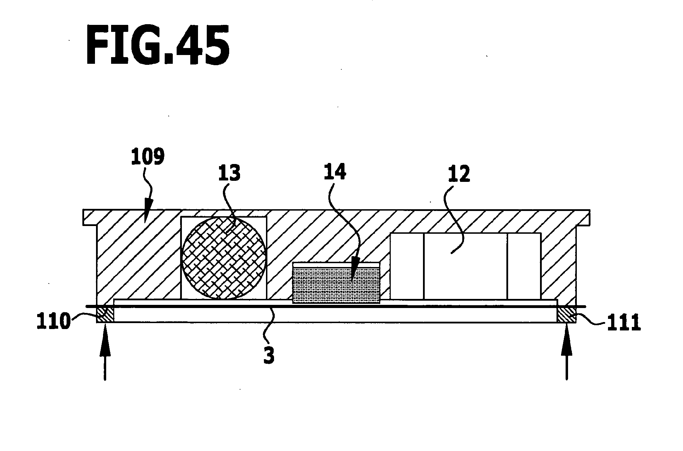

In den Figuren 44 und 45 ist eine weitere Ausgestaltungsmöglichkeit für ein starres Gehäuse 2 dargestellt. Das starre Gehäuse 2 ist dabei wie beim Ausführungsbeispiel der Figur 23 mit einem zentral und senkrecht von unten einmündenden Rohrstutzen 94 und mit einem horizontal und radial austretenden Rohrstutzen 95 versehen und an der Oberseite offen. In die oben offene Oberseite ist ein Einsatz 109 eingesetzt, der das Gehäuse 2 an der Oberseite dicht verschließt. Der Einsatz 109 nimmt in ähnlicher Weise wie das Übertragungsmittel 5 beim Ausführungsbeispiel der Figur 5 den Mikrochip 14 sowie Elektronik 12 und Elektronik 13 auf. An seiner Unterseite weist der Einsatz 109 einen ebenen, umlaufenden Rand 110 auf, der längs seiner Außenkontur verläuft und nur sehr geringfügig nach unten übersteht. Auf diesen ebenen Rand 110 ist die ebene, als dünne Metallfolie ausgebildete Membran 3 flächig aufgelegt und zwischen dem Einsatz 109 einerseits und einem ringförmigen Anlageelement 111 andererseits eingespannt, das dem Rand 110 gegenüberliegt und außenseitig mit diesem abschließt.

Im Bereich des Anlageelementes 111 und des Randes 110 sind der Einsatz 109 und die Membran 3 miteinander verlötet oder verschweißt.

Zur Herstellung dieser Verbindung wird zunächst bei dem Einsatz 109 vor dessen Einbringen in das Gehäuse 2 die Membran 3 flächig am Rand 110 anliegend angeordnet und durch das Anlageelement 111 gegen den Rand 110 gedrückt, und zwar durch eine Anpresskraft K (Figur 45). Dabei wird die Abmessung der Membran so gewählt, dass diese seitlich geringfügig über die Außenkontur des Einsatzes 109 und des Anlageelementes 111 vorsteht, wie dies aus der Figur 45 deutlich wird. In diesem Bereich des geringfügig überstehenden Randes der Membran 3 erfolgt die Verlötung oder Verschweißung mit dem Einsatz 109 und dem Anlageelement 111. Insbesondere bei einem Verschweißvorgang kann dabei der überstehende Randbereich der Membran beim Verschweißen entfernt werden, so dass ein bündiger Abschluss der Membran 3 mit dem Einsatz 109 und dem Anlageelement 111 erreicht werden kann.

Diese Baueinheit mit der aufgeschweißten oder aufgelöteten Membran 3 wird dann in das oben offene Gehäuse 2 eingesetzt und dieses dadurch abgedichtet.

Der freibleibende Innenraum des Gehäuses 2 bildet dann die Flüssigkeitskammer 84 aus, durch die die Hirnflüssigkeit hindurchfließt, deren Druck bestimmt werden soll.

**Patentansprüche**

1. Implantierbare Vorrichtung zur Erfassung von intracraniellen Drücken, wobei eine Druckmesseinrichtung verwendet wird, die in Wirkverbindung mit einem Sensor für eine telemetrische Messwertübertragung steht, wobei die Druckmesseinrichtung auf einem Mikrochip (4, 14) angeordnet und in diesen integriest ist, wobei sich der Microchip in einem starren Gehäuse (1; 17; 33) befindet und wobei die Druckübertragung von außen nach innen durch eine sehr dünne, biokompatible Membrane (3; 21) erfolgt, deren druckabhängige Bewegung über ein Übertragungsmittel (5) auf die Druckmesseinrichtung einwirkt, **dadurch gekennzeichnet, dass** als Übertragungsmittel (5) Luft oder ein spezielles Gas oder eine Flüssigkeit verwendet wird, mit dem eine Kammer (2; 15, 20, 22; 83) im Inneren des starren Gehäuses (1; 17; 33) gefüllt ist und dass die dünne, biokompatible Membran (3; 21) aus Metall besteht.

2. Vorrichtung nach Anspruch 1, **dadurch gekennzeichnet, dass** die Membran (3; 21) aus Titan oder einer Titanlegierung besteht.

3. Vorrichtung nach einem der voranstehenden Ansprüche, **dadurch gekennzeichnet, dass** die Membran (3; 21) eine Dicke von weniger als 0,05 Millimeter, vorzugsweise von weniger als 0,01 Millimeter, noch weiter bevorzugt von etwa 0,005 Millimeter aufweist.

4. Vorrichtung nach einem der voranstehenden Ansprüche, **dadurch gekennzeichnet, dass** die Membrane (3; 21) je nach Ausführungsform eine nachgiebige Fläche von etwa 100 mm$^2$ (Figur 22) oder 1 mm$^2$ (Figur 7), noch weiter bevorzugt von 4 mm$^2$ aufweist.

5. Vorrichtung nach einem der voranstehenden Ansprüche, **dadurch gekennzeichnet, dass** die Membrane (3; 21) mit dem Gehäuse (1; 17; 33) verschweißt ist.

6. Vorrichtung nach Anspruch 5, **dadurch gekennzeichnet, dass** die Membrane (3; 21) mit einem Blechrahmen (16) versehen ist und mit diesem am Gehäuse (1; 17) verschweißt ist.

7. Vorrichtung nach Anspruch 1, **dadurch gekennzeichnet, dass** die Membrane (3; 21) einstückig mit dem Gehäuse (1) ausgebildet ist.

8. Vorrichtung nach einem der voranstehenden Ansprüche, **dadurch gekennzeichnet, dass** die Membrane (3) unterschiedlich dicke Bereiche aufweist.

9. Vorrichtung nach Anspruch 8, **dadurch gekennzeichnet, dass** die Membrane (3) in ihren Randbereichen dicker ist als in ihrem zentralen Bereich.

10. Vorrichtung nach einem der voranstehenden Ansprüche, **dadurch gekennzeichnet, dass** ein Gas aus der Gruppe der Edelgase verwendet wird.

11. Vorrichtung nach einem der voranstehenden Ansprüche, **dadurch gekennzeichnet, dass** der Gehäuseinnenraum (2) bis auf das vorgesehene Gas- oder Flüssigkeitsvolumen mit einem Gas verdrängenden Füllmaterial (18) ausgefüllt ist.

12. Vorrichtung nach Anspruch 11, **dadurch gekennzeichnet, dass** das Füllmaterial (18) Kunststoff, Keramik oder ein metallisches Material ist.

13. Vorrichtung nach einem der Ansprüche 11 oder 12, **dadurch gekennzeichnet, dass** das Füllmaterial (18) an den drucksensiblen Flächen des Mikrochips (4) eine kleinstvolumige Druckkammer (22) nebst Zuleitungskanal (15) freilässt und dass das Füllmaterial (18) unterhalb der Membran (3) einen Gehäusehohlraum (20) als Druckkammer freilässt und dass die beiden Druckkammern (20, 22) durch eine kleinvolumige Leitung (15) verbunden sind.

14. Vorrichtung nach einem der voranstehenden Ansprüche, **dadurch gekennzeichnet, dass** das starre Gehäuse (1) eine außenseitig an die Membran (3) anschließende, abgeschlossene Flüssigkeitskammer (84) aufweist, die mit einer Zufuhrleitung (86, 87; 94) für Flüssigkeit verbunden ist.

15. Vorrichtung nach Anspruch 14, **dadurch gekennzeichnet, dass** die Flüssigkeitskammer (84) zusätzlich eine Ableitungsleitung (95; 96) für Hirnflüssigkeit aufweist.

16. Vorrichtung nach einem der Ansprüche 14 oder 15, **dadurch gekennzeichnet, dass** das starre Gehäuse (1) die Form einer flachen Dose aufweist, mit einer oberen, den Mikrochip (4) und das Übertragungsmittel (5) aufnehmenden Messkammer (83) und einem unteren, die Flüssigkeitskammer (84) ausbildenden Bereich.

**Claims**

1. Implantable device for determining intracranial pressures, wherein a pressure measuring device is used, which is operatively connected to a sensor for a telemetric measured value transfer, the pressure measuring device being arranged on a microchip (4, 14) and integrated therein, the microchip being located in a rigid housing (1; 17; 33), and the pressure transfer from the outside inwards occurring through a very thin, biocompatible membrane (3; 21), the pressure-dependent movement of which acts on the pressure measuring device via a transfer medium (5), **characterized in that** there is used as transfer medium (5) air or a special gas or a liquid, with which a chamber (2; 15, 20, 22; 83) inside the rigid housing (1; 17; 33) is filled, and **in that** the thin, biocompatible membrane (3; 21) is made of metal.

2. Device according to claim 1, **characterized in that** the membrane (3; 21) is made of titanium or a titanium alloy.

3. Device according to one of the preceding claims, **characterized in that** the membrane (3; 21) has a thickness of less than 0.05 millimetres, preferably of less than 0.01 millimetres, and further preferred of about 0.005 millimetres.

4. Device according to one of the preceding claims, **characterized in that** depending on the embodiment, the membrane (3; 21) has a flexible surface of about 100 mm$^2$ (Figure 22) or 1 mm$^2$ (Figure 7), and further preferred of 4 mm$^2$.

5. Device according to one of the preceding claims, **characterized in that** the membrane (3; 21) is welded to the housing (1; 17; 33).

6. Device according to claim 5, **characterized in that** the membrane (3; 21) is provided with a sheet metal frame (16) and is welded with this to the housing (1; 17).

7. Device according to claim 1, **characterized in that** the membrane (3; 21) is configured in one piece with the housing (1).

8. Device according to one of the preceding claims, **characterized in that** the membrane (3) has regions of different thickness.

9. Device according to claim 8, **characterized in that** the membrane (3) is thicker in its edge regions than in its central region.

10. Device according to one of the preceding claims, **characterized in that** a gas from the group of noble gases is used.

11. Device according to one of the preceding claims, **characterized in that** the housing interior (2) is filled with a gas-displacing filler material (18) except for the provided volume of gas or liquid.

12. Device according to claim 11, **characterized in that** the filler material (18) is plastic, ceramic or a metal material.

13. Device according to claim 11 or 12, **characterized in that** the filler material (18) leaves a minimum-volume pressure chamber (22) including supply duct (15) free on the pressure-sensitive faces of the microchip (4), and **in that** the filler material (18) leaves a housing cavity (20) as pressure chamber free below the membrane (3), and **in that** the two pressure chambers (20, 22) are connected by a small-volume conduit (15).

14. Device according to one of the preceding claims, **characterized in that** the rigid housing (1) has a closed fluid chamber (84), which adjoins the membrane (3) on the outside and is connected to a supply conduit (86, 87; 94) for fluid.

15. Device according to claim 14, **characterized in that** the fluid chamber (84) additionally has a drainage pipe (95; 96) for brain fluid.

16. Device according to claim 14 or 15, **characterized in that** the rigid housing (1) has the shape of a shallow can with an upper measurement chamber (83) receiving the microchip (4) and the transfer medium (5) and a lower region forming the fluid chamber (84).

## Revendications

1. Dispositif implantable pour relever des pressions intracrâniennes, en utilisant un système de mesure de pression, qui est en liaison interactive avec un capteur pour une transmission télémétrique de valeur de mesure, le système de mesure de pression étant agencé sur une micro-puce (4, 14) et intégré à celle-ci, la micro-puce se trouvant dans un boitier rigide (1; 17; 33), et la transmission de la pression de l'extérieur vers l'intérieur s'effectuant par une membrane biocompatible (3; 21) très mince, dont le mouvement en fonction de la pression agit sur le système de mesure de pression par l'intermédiaire d'un moyen ou milieu de transmission (5),
**caractérisé en ce que** l'on utilise en guise de moyen ou milieu de transmission (5), de l'air ou un gaz spécial ou bien encore un liquide, dont on remplit une chambre (2; 15, 20, 22; 83) à l'intérieur du boitier rigide (1; 17; 33), et **en ce que** la membrane biocompatible mince (3; 21) est réalisée en métal.

2. Dispositif selon la revendication 1, **caractérisé en ce que** la membrane (3; 21) est réalisée en titane ou en un alliage de titane.

3. Dispositif selon l'une des revendications précédentes, **caractérisé en ce que** la membrane (3; 21) présente une épaisseur de moins de 0,05 millimètre, de préférence de moins de 0,01 millimètre, et de manière privilégiée d'environ 0,005 millimètre.

4. Dispositif selon l'une des revendications précédentes, **caractérisé en ce que** la membrane (3; 21) présente, suivant le mode de réalisation, une surface souple d'environ 100 mm$^2$ (figure 22) ou de 1 mm$^2$ (figure 7), et de manière privilégiée de 4 mm$^2$.

5. Dispositif selon l'une des revendications précédentes, **caractérisé en ce que** la membrane (3; 21) est soudée au boitier (1; 17; 33).

6. Dispositif selon la revendication 5, **caractérisé en ce que** la membrane (3; 21) est munie d'un cadre en tôle (16) par l'intermédiaire duquel elle est soudée au boitier (1; 17).

7. Dispositif selon la revendication 1, **caractérisé en ce que** la membrane (3; 21) est réalisée d'un seul tenant avec le boitier (1).

8. Dispositif selon l'une des revendications précédentes, **caractérisé en ce que** la membrane (3) présente des zones d'épaisseur différente.

9. Dispositif selon la revendication 8, **caractérisé en ce que** la membrane (3) est plus épaisse dans la région de ses zones de bordure que dans sa zone centrale.

10. Dispositif selon l'une des revendications précédentes, **caractérisé en ce que** l'on utilise un gaz du groupe des gaz rares.

11. Dispositif selon l'une des revendications précédentes, **caractérisé en ce que** l'espace intérieur (2) du boitier, est rempli, exception faite du volume prévu de gaz ou de liquide, d'un matériau de remplissage (18) refoulant le gaz.

12. Dispositif selon la revendication 11, **caractérisé en ce que** le matériau de remplissage (18) est une matière plastique, une céramique ou un matériau métallique.

13. Dispositif selon l'une des revendications 11 ou 12, **caractérisé en ce que** le matériau de remplissage (18) laisse dégagé, au niveau des surfaces sensibles à la pression de la micro-puce (4), outre un canal d'arrivée (15), une chambre de pression (22) de volume très faible, et **en ce que** le matériau de remplissage (18) laissé dégagé, sous la membrane (3), une cavité de boitier (20) en tant que chambre de pression, et **en ce que** les deux chambres (20, 22) sont reliées par une conduite (15) de faible volume.

14. Dispositif selon l'une des revendications précédentes, **caractérisé en ce que** le boitier rigide (1) présente une chambre de liquide (84) fermée, qui se raccorde du côté extérieur à la membrane (3), et est reliée à une conduite d'alimentation (86, 87; 94) pour du liquide.

15. Dispositif selon la revendication 14, **caractérisé en ce que** la chambre de liquide (84) présente en supplément une conduite d'évacuation (95; 96) pour du liquide cérébro-spinal.

16. Dispositif selon l'une des revendications 14 ou 15, **caractérisé en ce que** le boitier rigide (1) présente une forme de boite plate comprenant une chambre de mesure supérieure (83), qui reçoit la micro-puce (4) et le moyen ou milieu de transmission (5), et une zone inférieure formant la chambre de liquide (84).

# FIG.1

# FIG.2

# FIG.3

# FIG.4

# FIG.5

# FIG.5a

# FIG.6

# FIG.7

# FIG.8

16

# FIG.9

17

21

24

16

19

# FIG.10

# FIG.11

# FIG.12

EP 2 025 286 B1

# FIG.13

EP 2 025 286 B1

# FIG.14

# FIG.15

# FIG.16

# FIG.17

FIG.18

FIG.19

# FIG.20

# FIG.21

# FIG.22

EP 2 025 286 B1

# FIG.23

# FIG.24

# FIG.25

EP 2 025 286 B1

# FIG.26

EP 2 025 286 B1

# FIG.27

FIG.28

# FIG.29

# FIG.30

EP 2 025 286 B1

# FIG.31

EP 2 025 286 B1

# FIG.32

# FIG.33a

# FIG.33b

# FIG.34a

# FIG.34b

# FIG.35a

# FIG.35b

# FIG.36a

**41**   **4**

**1**   **3**

# FIG.36b

**41**   **4**

**1**   **3**

# FIG.37a

# FIG.37b

# FIG.38

**4**  **58**

**1**  **3**  **101**

# FIG.39

**F**  **101**  **4**

# FIG.40

# FIG.41

# FIG.42

# FIG.43

104  41          4              58

1        3       107

# FIG.44

F

104    4

107

# FIG.45

# FIG.46

## IN DER BESCHREIBUNG AUFGEFÜHRTE DOKUMENTE

**In der Beschreibung aufgeführte Patentdokumente**

- DE 19638813 C1
- US 4738267 A
- DE 19705474
- US 6113553 A
- US 4676255 A
- DE 19858172
- DE 10156494
- EP 1312302 A2
- US 3946724 A